# EUROPEAN PATENT SPECIFICATION

(11) **EP 2 054 101 B1**
(45) Date of publication and mention of the grant of the patent: **11.01.2012**
(21) Application number: 07836532.7
(22) Date of filing: 03.08.2007
(51) Int. Cl.: A61L 31/08, A61L 27/28

(54) **IMPLANTABLE MEDICAL DEVICE WITH PARTICULATE COATING**
IMPLANTIERBARE MEDIZINISCHE VORRICHTUNG MIT PARTIKELBESCHICHTUNG
APPAREIL MÉDICAL IMPLANTABLE AVEC REVÊTEMENT PARTICULAIRE

(30) Priority: 03.08.2006 US 835400 P
(43) Date of publication of application: 06.05.2009
(73) Proprietor: Cook Medical Technologies LLC, Bloomington, IN 47402 (US)
(72) Inventor: GREWE, David, D., West Lafayette, IN 47906 (US); VOORHEES, William, D., West Lafayette, IN 47906 (US); RAGHEB, Anthony, O., West Lafayette, IN 47906 (US)
(74) Representative: Garratt, Peter Douglas
(86) International application number: PCT/US2007/017431
(87) International publication number: WO 2008/019116

(56) References cited:
- EP-A- 0 595 606
- WO-A-2004/027385
- WO-A-2006/104894
- WO-A-2007/056372
- US-A1- 2003 069 638

## Description

### Technical Field

The present invention relates to implantable medical devices comprising a vessel-engaging surface useful to secure the medical device within a body vessel, as well as methods of using and manufacturing the coated medical devices.

### Background of the Invention

Various implantable medical devices can be deployed within the lumen of a body vessel using minimally-invasive transcatheter techniques. For example, implantable medical devices can function as a stent, a shunt, or a replacement valve. Such devices can include an expandable frame configured for implantation in the lumen of a body vessel, such as an artery or a vein. Minimally invasive techniques and instruments for placement of intralumenal medical devices have been developed to treat and repair undesirable conditions by implantation of the medical devices within body vessels. Intralumenal medical devices can be introduced to a point of treatment within a body vessel using a delivery catheter device passed through the blood vessels communicating between a remote introductory location and the implantation site, and released from the delivery catheter device at the point of treatment within the body vessel. Intralumenal medical devices can be deployed in a vessel at a point of treatment, the delivery device withdrawn from the vessel, and the medical device retained within the vessel to provide sustained improvement in vascular function or to increase vessel patency. Implantable medical devices can desirably comprise features that secure the implantable device within the body vessel upon deployment.

For some medical applications, multiple implantable medical devices can be implanted within a body vessel at a static orientation and distance with respect to each other. For example, a series of two or more stents can be implanted in a femoral artery with a desired distance or orientation between the stents. Movements of the artery proximate to the implanted stents can cause the stents to migrate, undesirably altering the preferred relative longitudinal position of the stents relative to one another.

Numerous methods and device modifications have been proposed to secure implantable medical devices in place in the body. In one approach, the medical device is anchored mechanically to biological tissue. Medical devices can be anchored to the surrounding tissues by physical and mechanical means (e.g., screws, cements and porous surfaces) or by friction. For example, mechanical attachment of a device to the site can be effected by using a fastener, such as a suture or staple. In another approach, the device includes in its design mechanical means for fastening it into the surrounding tissue. For example, the device may include metallic spikes, anchors, hooks, barbs, pins, clamps, or a flange or lip to affix the device in place (see, e.g., U.S. Pat. Nos. 4,523,592; 6,309,416; 6,302,905; and 6,152,937). Methods for preventing device migration have also included mechanically altering the surface characteristics of the device. One such approach involves scoring or abrading the surface of the implant. The roughened surfaces promote cell, bone or tissue adhesion for better affixing of the implants in the body (see, e.g., WO 96/29030A1). Another approach provides medical device coatings comprising silk to induce the production of fibrous (scar) tissue to anchor the medical device upon implantation within the body vessel (e.g., published US patent application US 2005/0186247, filed December 7, 2004). US2004/0199241 A1 (Gravett et al.) discloses silk-containing stent grafts comprising an endoluminal medical device and a graft, where the silk induces the adhesion of the stent graft to the vessel walls. US 2006/0178739 A1 (Shalaby et al.) discloses endourological stents formed from fiber-reinforced elastomeric films configured to prevent migration of the device from the implantation site. US 5,059,209 (Jones) discloses a prosthetic device prepared by curing a mixture of a particulate inorganic solid with polymer. US 2007/0106363 A1 (Weber) describes implantable medical devices including at least one particle-containing region whose surface is at least partially coated with a diamond-like coating.

However, there remains a need for an effective, long-lasting and biocompatible approach for securing implantable medical devices into or onto biological tissue within a body vessel.

### Summary of the Invention

The present disclosure relates to particulates medical device costings for an implantable medical device. The coatings are useful for preventing emigration of the medial device upon implantation of the medical device in a body vessel Examples of implantable medical device include an expandable stent, a portion of a prosthetic valve or indwelling catheter. The particulate coating preferably, comprises a plurality of particles affixed to an outer surface of the medical device. The particles preferably include a rigid biocompatible inorganic material and may be configured to engage the will of a body vessel upon placement of a medical device within the body vessel. The particles are preferably formed from a sufficiently rigid material, such as one or more materials selected from the group consisting of: aluminum oxide, silicon carbide, hydroxyapatite, silicon dioxide, iron oxide, alumina zirconia, silicon carbide, alumina zirconia and emery.

The particles are desirably secured two the medical device by any suitable method. In one embodiment, the particulate coating comprises a plurality of rigid particles embedded a carrier coated on a medical device frame, such as a granular coating. The carrier can include a biodegradable polymers, a biostable polymer such as a polyurethane or an extracellular matrix material. The particles can be applied together with a carrier composition or can be applied separately to a coating comprising the carrier. The particulates coating can be applied to any suitable medical device. In one embodiment, the particulate coating is applied to an exterior surface of an implantable stent, such as a self-expanding frame or a tubular plastic stent. Another embodiment provides a particulate coating comprising a plurality of rigid particles embedded in a mesh fabric to form a particulate-coated graft material. Particulate coatings are preferably configured to engage the wall of a body vessel upon implantation of the medical device therein, Another embodiment provides a particulate coating that is free of a carrier, such as inorganic particles attached to the surface of a medical device by ion beam implantation and/or sputter deposition.

Forming layers containing particles is generally known. For example, it has also been disclosed in EP 0 595 606 to spread a particle-containing or particle-forming liquid over the surface of a high density liquid. In the disclosed method, the particles are aggregated two-dimensionally by reducing the thickness of the particle-containing or particle-forming liquid and the aggregated and thin two-dimensional particulate film formed is brought into contact with the surface of a solid substrate to transfer and fix said thin film thereon. The two-dimensional aggregation can take place to form thin films of high quality.

WO2004/027385 discloses a device having a surface and a functional layer associated with the surface, where the functional layer includes particles having a structure substituted with a functional group, where the functional group is adapted to modify a property of the device, the device is sufficiently biocompatible for application to a multicellular organism and the particles have an average diameter of about 5 nm to about 10 microns.

The use of calcium phosphate powder in making bone implants is known from US 2003/069638. The process involves selectively fusing layers of calcium powders that have been coated or mixed with polymer binders. The calcium powder mixture may be foamed into layers and the polymer fused with a laser. Complex three-dimensional geometrical shapes can be automatically replicated or modified using this approach.

In addition, WO2007/056372, which belongs to the state of the art under Art.54(3) EPC, discloses various medical devices, including implantable or insertable medical devices, are provided, which comprise at least one particle-containing region whose surface is at least partially coated with a diamond- like coating.

WO2006/104894, which also belongs to the state of the art under Art.54(3) EPC, discloses coatings for implantable devices or endoluminal prosthesis, such as stents, including a method of forming the coatings. The coatings can be used for the delivery of an active ingredient or a combination of active ingredients.

The implantable medical device preferably includes a frame expendable from a compressed state for delivery via transcatheter implantation and a radially expanded state for deployment from a catheter within a body vessel. Alternatively, the medical device can be configured as a tube, such as a biliary drainage stent, formed from a thermoplastic material with or without drainage holes along the sides of the tube. Preferably, the medical devices are configured as endolumenally implantable valves, shunts, catheters, stent grafts or stents. Implantable valves and stent grafts can include a radially expandable frame and a material attached to the frame. The particulate coating can be applied to the frame and/or the graft material. In an implantable valve, the material can form one or more moveable valve leaflets for regulating the flow of fluid within a body vessel. In a stent graft, a material can be attached around the outer surface of the implantable frame. Optionally, the particulate coating can be embedded in a remodelable material, such as small intestine submucosa (SIS), to form a graft material or a frame coating.

For example, in one aspect, a medical device comprising a radially expandable frame adapted for implantation in a body vessel is provided where the frame defining a substantially tubular lumen and having an exterior surface, wherein at least a portion of the exterior surface comprises a vessel-engaging surface formed by a plurality of inorganic particles attached to the exterior surface to form the vessel-engaging surface with an average roughness of about 10 nm and 100 micrometers and consisting of one or more materials selected from the group consisting of: aluminum oxide, silicon carbide, hydroxyapatite, silicon dioxide, iron oxide, alumina zirconia, silicon carbide, alumina zirconia, emery, stainless steel, cobalt-chromium, nitinol and tantalum. The vessel-engaging surface may have a maximum roughness of less than about 25 micrometers and a concentration of inorganic particles of at least 10,000 per square centimeter of the vessel-engaging surface. The inorganic particles may consist of aluminum oxide.

The present disclosure also describes methods of securing a medical device having a vessel-engaging surface within a body vessel. The vessel-engaging surface is preferably an abrasive exterior surface having a particulate coating comprising a plurality of abrasive inorganic particles extending from the surface. The particulate coating may have a surface roughness and hardness suitable to engage the wall of the body vessel, such as an average surface roughness of between about 10 nm and 100 micrometers and having a hardness suitable to engage the wall of a body vessel, such as a hardness of at least about 30 - 100 HRD on the "D" scale measured by the ASTM D2240 type D standard, and preferably about 30 - 80, 40 - 80, 50 - 80, 60 - 80 or about 70 on the "D" scale. Accordingly, a method of treatment may include deployment of such a radially-expandable medical device within a body vessel from a catheter delivery system. For example, a medical device including a balloon-expandable support frame may be radially expanded from a balloon catheter having an expandable balloon, or a self-expanding frame from a catheter that does not include a balloon. The method may include inserting a portion of the catheter delivery system including the medical device into a body vessel, advancing the balloon within the body vessel to a treatment site, radially expanding the medical device to directly contact the vessel-engaging surface with an inner wall of the body vessel, and removing the catheter delivery system from the body vessel while retaining the medical device within the body vessel.

In one aspect, the invention provides a vessel-engaging medical device comprising a radially expandable medical device adapted for implantation in a body vessel, the medical device defining a substantially tubular lumen and having an exterior surface facing radially opposite the tubular lumen, wherein at least a portion of the exterior surface comprises a vessel-engaging surface formed by a plurality of inorganic particles attached to the exterior surface to form the vessel-engaging surface with an average roughness of about 10 nm and 100 micrometers, the inorganic particles consisting of one or more materials selected from the group consisting of: metals, metal alloys and inorganic oxides.

In another aspect of the present invention, a method of making an implantable medical device comprises providing a frame having one or more projections with at least one sharp edge. The method further comprises the step of applying a particulate coating to the at least one surface at a desired thickness. In yet another aspect of the present invention, a method of treating a subject comprises implanting a medical device at a point of treatment, wherein the medical device comprises a frame and a particulate coating over at least one sharp edge of a projection from the frame.

Additional understanding of the invention can be obtained by referencing the detailed description of embodiments of the invention, below, and the appended drawings.

### Brief Description of the Drawing

Figure 1 is a side view of an implantable frame including a particulate coating on the distal and proximal portions of the frame;
Figure 2 is detailed view of a portion of the particulate coating on the implantable frame in Figure 1.
Figure 3 is an exploded view of the assembly of a stent graft comprising a particulate coating on the distal and proximal ends of the graft material.
Figure 4 is a longitudinal cross sectional view of a body vessel having a pair of the implantable frames shown in Figure 1 implanted at a fixed longitudinal distance from each other.
Figure 5 is a portion of a transcatheter endolumenal delivery system comprising the Implantable frame in Figure 1 in a radially compressed state within the distal portion of a delivery catheter.

### Detailed Description

The following detailed description and appended drawings describe and illustrate various exemplary embodiments of the invention. The description and drawings serve to enable one skilled in the art to make and use the invention, and are not intended to limit the scope of the invention in any manner.

The invention provides medical devices for implantation in a body vessel, methods of making the medical devices, and methods of treatment that utilize the medical devices.

As used herein the terms "comprise(s)," "include(s)," "having," "has," "contain(s)," and variants thereof, are intended to be open-ended transitional phrases, terms, or words that do not preclude the possibility of additional acts or structure.

As used herein, the term "body vessel" means any tube-shaped body passage lumen that conducts fluid, including but not limited to blood vessels such as those of the human vasculature system, biliary ducts and ureteral passages.

As used herein, the term "Implantable" refers to an ability of a medical device to be positioned at a location within a body, such as within a body vessel. Furthermore, the terms "implantation" and "implanted" refer to the positioning of a medical device at a location within a body, such as within a body vessel.

As used herein, the term "inorganic" refers to a material that includes a non-carbon element, such as silicon, iron or calcium and various oxides thereof.

As used herein, "endolumenally," "intralumenally" or "transluminal" all refer synonymously to implantation placement by procedures wherein the prosthesis is advanced within and through the lumen of a body vessel from a remote location to a target site within the body vessel. In vascular procedures, a medical device will typically be introduced "endovascularly" using a catheter over a wire guide under fluoroscopic guidance. The catheters and wire guides may be introduced through conventional access sites to the vascular system, such as through the femoral artery, or brachial and subclavian arteries, for access to the coronary arteries.

A "biocompatible" material is a material that is compatible with living tissue or a living system by not being unduly toxic or injurious and without causing an unacceptable degree of inflammatory reaction.]

The term "frame" refers to any biocompatible frame suitable for implantation within a body vessel. Preferably, a frame can expand from a compressed, or unexpanded, delivery configuration to one or more radially expanded deployment configurations, for example through self-expansion or balloon expansion of the frame. The expanded configuration can have any suitable cross-sectional configuration, including circular or elliptical. In one embodiment, the frame can be oriented along the longitudinal axis of a body vessel in the expanded or compressed configurations.

As used herein, a "point of attachment" refers to a location wherein a bond is formed thereby linking two adjacent surfaces.

The terms "remodelable" or "bioremodelable" refer to the ability of a material to allow or induce host tissue growth, proliferation or regeneration following implantation of the tissue in vivo. Remodeling can occur in various microenvironments within a body, including without limitation soft tissue, a sphincter muscle region, body wall, tendon, ligament, bone and cardiovascular tissues. Upon implantation of a remodelable material, cellular infiltration and neovascularization are typically observed over a period of about five days to about six months or longer, as the remodelable material acts as a matrix for the ingrowth of adjacent tissue with site-specific structural and functional properties. The remodeling phenomenon which occurs in mammals following implantation of submucosal tissue includes rapid neovascularization and early mononuclear cell accumulation. Mesenchymal and epithelial cell proliferation and differentiation are typically observed by one week after in vivo implantation and extensive deposition of new extracellular matrix occurs almost immediately. In some embodiments, fluid contacting autologous cells on an implanted remodelable material can affect the growth of autologous tissue on the implanted remodelable material.

### PARTICULATE COATINGS

The present disclosure relates to medical devices for implantation in a body vessel. More specifically, preferred embodiments relate to a medical device comprising a particulate coating on at least a portion of a surface configured to contact a body vessel upon implantation of the medical device within the body vessel. The particulate coating is preferably a plurality of particulates attached to a portion of the -medical device in a manner permitting the particulates to engage the wall of a body vessel in a manner effective to secure the medical device in the body vessel upon implantation. The terms "particulate" and "particles" are used interchangeably, unless otherwise indicated. The particles are preferably dimensioned to abrasively engage the wall of a body vessel to prevent movement of an implanted medical device. Accordingly, the particles may have a high aspect ratio, and may be substantially needle-like. The particles may also have a triangular, substantially square, substantially rectangular, substantially elliptical, or substantially semi-circular cross-sectional shape. Preferably, the particulate coatings form a vessel-engaging surface on an implantable medical device.

A variety of particulate coating materials may be used to form an abrasive particulate coating on an exterior surface of a medical device. Preferably, the particulate coating is durable, solid, and rigid at room temperature, and when exposed to blood under normal blood temperatures and pH. The particulate composition comprises a plurality of particles having a rigidity and shape adapted to engage the wall of a body vessel. The particles can be derived from natural or synthetic minerals that are crushed, then cleaned and graded for size. Abrasive minerals are crushed from larger into smaller pieces, then are screened and graded based on the size of the grain, called grit. The larger grains can be sifted out using screens made of fine silk threads. The finer grits are separated by blowing through the air, centrifugal methods or by sedimentation in water. The particulate composition can be in the form of microspheres (porous or non-porous), microparticles, and/or nanoparticles.

The particles may be formed from any material having a hardness suitable to engage the wall of a body vessel. Preferably, the particle has a hardness of about 70 HRD on the "Rockwell D" scale. For comparison, polyethylene tests at about 30 HRD, and plexiglass is between 70 and 75 HRD on the "Rockwell D" scale (ASTM D2240, Vol. 0991). The vessel-engaging projection preferably has a hardness of at least 30 HRD on the "D" scale, particularly at least 50 on the "D" scale, more particularly above 60 HRD on the "D" scale, and most particularly about 70 HRD or above on the "D" scale. A hardness of 60-70 is usable in many applications. Hardness testing may be done using an e2000 series durometer from PTC Instruments (model 502D). The durometer preferably conforms to the ASTM D2240 type D standard. Indentors conforming to ASTM D2240 type D is a sharp point indentor with a 30 degree included angle and applies a force of 4536 gmf on the sample being tested.

Any suitable particulate size can be selected. For instance, particles can range in size from sub-micron to up to 2 mm, depending on the desired role of the component chosen. In different embodiments, the size of the particles is between about 8 and about 300 micrometers or between about 1 5 and about 50 micrometers. The particles preferably have an average individual size of about 100 µm**,** 10 µm, 1 µm, 100 nm or 10 nm, or smaller in size. Preferably, the particles have an asymmetric, non-circular morphology. Particularly preferred particle morphologies are acicular (needle-shaped), with a length of about 10 nm - 50 nm (0.01-0.05 µm). The particles may be one or more inorganic solids in the form of ground or extruded particles, preferably wherein the particle size is from 90% thereof being less than 100 micrometers to 1.0 micrometer, preferably from 90% being less than 50 micrometers to 0.1 micrometer. Preferably a portion of the particles are of sub-micron size, more preferably about 10% thereof are such particles, e.g. of silica.

The particles are preferably formed from a biocompatible inorganic material. For example, the particles may be inorganic acicular particles with the desired 10-50 nm length can be purchased or prepared using a jet mill or ultrasonic nebulizer. These particulate compositions can be formed, for example, by spray-drying methods, milling methods, coacervation methods, W/O (water-oil) emulsion methods, W/O/W emulsion methods, and solvent evaporation methods. In some embodiments, these compositions can include microemulsions, emulsions, liposomes and micelles. Alternatively, such compositions may also be readily applied as a "spray", which solidifies into a film or coating for use as a device/implant surface coating or to line the tissues of the implantation site. Such spray methods may be used to prepare particulate coatings of microparticles of a wide array of sizes, including for example, from 100 nm to 3 micrometers, from 10 micrometers to 30 micrometers, and from 30 micrometers to 100 micrometers.

The particles preferably include an inorganic material comprising at least one non-carbon element. Suitable particles can be obtained from minerals such as aluminum oxide, silicon carbide and alumina zirconia. These minerals can be made by combining natural substances such as Bauxite clay and coke, from burning coal, in a large furnace and heating them to extremely high temperatures. The heating and specialized cooling process can take days, and the mineral that results can have a crystalline structure. Preferably, the particles of the particulate composition comprise materials selected from the group consisting of: silicon carbide, alumina zirconia, talc, mica, silica, a glass (e.g. a borosilicate), hydroxyapatite, silicone dioxide, iron oxide, aluminum oxide, emery, calcium phosphates, calcium carbonates and calcium sulfates. An aluminum oxide is desirably selected from the group consisting of: premium aluminum oxide, white aluminum oxide and brown aluminum oxide. An aluminum zirconia is preferably prepared by dissolving a zirconium oxide in aluminum oxide at molten temperatures. Premium aluminum oxide is desirably prepared by thermally treating fused aluminum oxide to improve hardness and friability properties. Emery may be an aluminum oxide with a reduced ferric oxide content. Another useful natural mineral source for the particles is garnet, a naturally occurring mineral. Hydroxy apatite particles include those described, e.g., in U.S. Pat. No. 5,009,898. The particles may be formed of other inorganic materials that are metals, metal salts or metal ions. The metal ions may include silver, copper, zinc, mercury, tin, lead, bismuth, cadmium, chromium and thallium ions. The inorganic metal salts may include silver acetate, silver benzoate, silver carbonate, silver ionate, silver iodide, silver lactate, silver laureate, silver nitrate, silver oxide, silver palpitate, silver protein, and silver sulfadiazine. The particles may also include antimicrobial zeolites prepared by replacing all or part of the ion-exchangeable ions in zeolite with ammonium ions and antimicrobial metal ions, as described in U.S. Pat. Nos. 4,938,958 and 4,911,898. The particles may include ceramic particles such as zeolites, zirconium phosphates or other ion-exchange ceramics. Zirconium phosphates are described, e.g., in U.S. Pat. Nos. 5,296,238; 5,441,717; and 5,405,644. Inorganic particles, such as the oxides of titanium, aluminum, zinc and copper, may be used to form the coating. Still other suitable inorganic soluble glass particles are described, e.g., in U.S. Pat. Nos. 5,766,61 and 5,290,544.

The particulate vessel-engaging surface may also be formed from, combined with or embedded in biodegradable materials that are sufficiently rigid, preferably formed in a needle-like morphology. Representative examples of suitable biodegradable polymers suitable include albumin, collagen, gelatin, hyaluronic acid, starch, cellulose and cellulose derivatives (e.g., methylcellulose, hydroxypropylcellulose, hydroxypropylmethylcellulose, carboxymethylcellulose, cellulose acetate phthalate, cellulose acetate succinate, hydroxypropylmethylcellulose phthalate), casein, dextrans, polysaccharides, fibrinogen, poly(ether ester) multiblock copolymers, based on poly(ethylene glycol) and poly(butylene terephthalate), tyrosine-derived polycarbonates (e.g., U.S. Pat. No. 6,120,491), poly(hydroxyl acids), poly(D,L-lactide), poly(D,L-lactide-co-glycolide), poly(glycolide), poly(hydroxybutyrate), polydioxanone, poly(alkylcarbonate) and poly(orthoesters), polyesters, poly(hydroxyvaleric acid), polydioxanone, poly(ethylene terephthalate), poly(malic acid), poly(tartronic acid), poly(acrylamides), polyanhydrides, poly(ester-amides), poly(ester-imides), poly(ester-ureas), poly(ester-urethane-ureas), poly(anhydride-esters), poly(anhydride-imides), polyphosphazenes, poly(amino acids), poly(alkylene oxide)-poly(ester) block copolymers (e.g., X--Y, X--Y--X or Y--X--Y, where X is a polyalkylene oxide and Y is a polyester (e.g., PLGA, PLA, PCL, polydioxanone and copolymers thereof), and copolymers as well as blends thereof. (see generally, Ilium, L., Davids, S. S. (eds.) "Polymers in Controlled Drug Delivery" Wright, Bristol, 1987; Arshady, J. Controlled Release 17: 1-22, 1991; Pitt, Int. J. Phar. 59: 173-196, 1990; Holland et al., J. Controlled Release 4: 155-0180, 1986).

In some embodiments, the particulate composition is combined with a carrier that adheres the particles of the particulate composition to the medical device, and maintains the particles in a desired position and orientation on a surface of the medical device. The carrier can be a polymer or non-polymer, and may include tissue or tissue-derived components such as extracellular matrix material. A coating composition comprising the carrier and the particulate composition may be applied to, or formed into, an implantable medical device. The coating composition preferably comprises 10-80%, more preferably 40-75% and particularly more preferably 65-70% by volume of a particulate inorganic solid. The particulate inorganic solid tends both to reinforce the composite and to enhance its stiffness.

For example, the carrier can be a biostable carrier polymer selected to be rigid enough to retain the particles in a desired orientation in a coating, while flexible enough to resist cracking or peeling during delivery of the medical device within a body vessel, or during dynamic forces on the medical device after implantation. A particulate composition may be admixed with a polymeric composition (which may be either biodegradable or non-biodegradable) or non-polymeric composition. The polymeric or non-polymeric composition (i.e., carrier) can be used to coat the device or as a component of the materials used to manufacture the device. Preferably, the carrier polymer is biostable, and does not substantially dissipate upon implantation within the body vessel. Representative examples of non-degradable polymers suitable for combination with a particulate include poly(ethylene-co-vinyl acetate) ("EVA") copolymers, silicone rubber, acrylic polymers (e.g., polyacrylic acid, polymethylacrylic acid, polymethylmethacrylate, poly(butyl methacrylate)), poly(alkylcyanoacrylate) (e.g., poly(ethylcyanoacrylate), poly(butylcyanoacrylate), poly(hexylcyanoacrylate), and poly(octylcyanoacrylate)), polyethylene, polypropylene, polyamides (nylon 6,6), polyurethanes (including hydrophilic polyurethanes), poly(ester-urethanes), poly(ether-urethanes), poly(ester-urea), poly(carbonate urethane)s, polyethers (poly(ethylene oxide), poly(propylene oxide), polyoxyalkylene ether block copolymers based on ethylene oxide and propylene oxide such as PLURONICs and PLURONICs R and poly(tetramethylene glycol)), styrene-based polymers (polystyrene, poly(styrene sulfonic acid), poly(styrene)-block-poly(isobu- tylene)-block-poly(styrene), poly(styrene)-poly(isoprene) block copolymers], and vinyl polymers (polyvinylpyrolidone, poly(vinyl alcohol), poly(vinyl acetate phthalate) as well as copolymers and blends thereof. Polymers may also be developed which are either anionic (e.g., alginate, carrageenan, carboxymethyl cellulose, poly(acrylamido-2-methyl propane sulfonic acid) and copolymers thereof, poly(methacrylic acid) and copolymers thereof, and poly(acrylic acid) and copolymers thereof, as well as blends thereof) or cationic (e.g., chitosan, poly-L-lysine, polyethyleneimine, and poly(allyl amine) and blends thereof (see generally, Dunn et al., J. Applied Polymer Sci. 50: 353-365, 1993; Cascone et al., J. Materials Sci.: Materials in Medicine 5: 770-774, 1994; Shiraishi et al., Biol. Pharm. Bull. 16(11): 1164-1168, 1993; Thacharodi and Rao, Int'l J. Pharm. 120: 115-118, 1995; Miyazaki et al., Intl J. Pharm. 1 18: 257-263, 1995).

Preferably, the particulate inorganic solid may be further combined with a suitable coupling agent to improve the bonding of the particle to the carrier component. For example, where the particulate inorganic solid is silica it may be treated with a suitable silane coupling agent, e.g. .gamma.-methacryl-oxypropyltrimethoxysilane.

The coating may include vessel-engaging projections having a hardness suitable to engage the wall of the body vessel. For example, the vessel-engaging projections may have an arcuate morphology (e.g., a "fish hook" shape) or a straight, needle-like shape. The vessel-engaging projections can be formed from any suitable material, including portions of a metal frame or an extracellular matrix material, or a polymer coating. In one example, the projections are formed from a biopolymer, such as silk, plant-derived materials or other cellulose-containing polymers.

### MEDICAL DEVICE CONFIGURATIONS

In a first embodiment, medical devices comprise a particulate coating on an implantable frame. The particulate coating can be adhered to any portion of the medical device frame, but are preferably attached to an external surface to contact the wall of a body vessel upon implantation of the frame. Also preferably, the particulate coating is applied to the distal and proximal regions of the implantable frame. The particulate coating can be attached to the frame in any suitable manner.

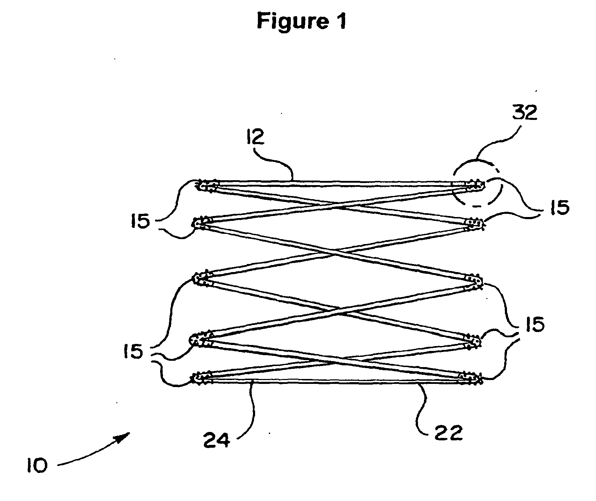

**Figure 1** is a perspective view of an implantable medical device 10 including a plurality coated regions 15 comprising a vessel-engaging region formed from a particulate coating. The medical device 10 is a stent including a radially expandable frame 12 shown in a radially expanded state. The medical device 10 includes a plurality of interconnected struts 20 and bends 32, and includes a pair of longitudinally joined serpentine ring members 22, 24. The outer surface of the frame 12 is adapted to contact a body vessel, and includes the plurality of struts and bends forming two longitudinally-connected sinusoidal hoops. The frame 12 can be formed from a self-expanding material, such as a superelastic nickel-titanium alloy, that exerts an outward radial force against the body vessel. The frame 12 is delivered in a radially compressed state by a delivery catheter and permitted to expand by self-expansion at the point of treatment.

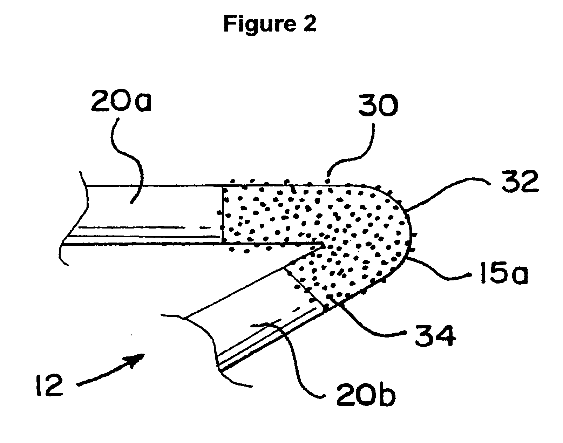

Referring to **Figure 2**, a detail of a first vessel-engaging region 15(a) of the frame 12 is shown. The vessel-engaging regions 15 are preferably configured as particulate regions to engage the interior wall of a body vessel to secure the frame 12 and prevent migration of the frame 12 therein. The particles can have any suitable size or cross-sectional shape. The vessel-engaging region 15(a) comprises a plurality of particles 30 embedded in a carrier material 34 adhered to the surface of a bend 32 between a first strut 20(a) and a second strut 20(b). Any suitable density of particles 30 can be adhered to the first vessel-engaging region 15(a). Preferably about 50-100 particles, or more, are present per square inch of a coated region (i.e., about 7.75 to 15.5 particles per square centimeter), most preferably at least about 80 particles/in² (i.e., about 12.4 particles per cm²).

The particulate coating of the vessel-engaging region(s) can be incorporated into the structure of the medical device by any suitable configuration, including: (1) directly attaching the particulate coating to one or more surfaces of an implantable medical device frame; (2) incorporating the particulate coating into the material forming the medical device (e.g., constructing the device from a mixture of the particulate and a carrier material); (3) covering portions of the medical device with a sleeve, cover or mesh containing the particulate coating (i.e., a covering comprised of a particulate mixed with a suitable carrier such as an extracellular matrix material, EVA, PLA, or polyurethanes); (4) impregnating the medical device with the desired particulate composition (e.g., by soaking a porous portion of a medical device in a suspension of the particulates); or (5) roughening a surface of the medical device (i.e., creating ridges or indentations) on all, or parts, of the device surface to produce a desirably particulate surface.

In a first aspect, the particulate composition is attached to a portion of a medical device frame to form a vessel-engaging region in any suitable manner, including: spraying the medical implant with particulate vessel-engaging surface and/or carrier (polymeric or non-polymeric) composition to create a coating on all, or parts of the internal or external surface of the medical device, (2) dipping the implant or device into a solution of particulate vessel-engaging surface and/or carrier (polymeric or non-polymeric) solution to coat all or parts of the device, (3) mechanically attaching the particles, or a composite material comprising the particles, to the medical device frame via an adhesive thermal, electrostatic, or ionic treatment, applying a non-particulate coating to the medical device and attaching the desired particulate vessel-engaging surface to the coating.

In a second aspect of the first embodiment, the particulate coating is formed by adhering a plurality of particles to a coating on the implantable frame that is adapted to retain the particles. The implantable frame may be coated with a suitable adhesive, which is then contacted with the particles in a manner effective to bind the particles thereto. For example, the device can be coated with a polyurethane and then allowed to partially dry such that the surface is still tacky. A particulate with or without another carrier can then be applied to all or a portion of the tacky coating after which the device is dried. Application of the particulate to a sticky, organic-coated frame can be performed by rolling the stent in the particulate or by blowing the particles onto the sticky coating. Where the particulate composition has an acicular morphology, the particles may subsequently be aligned in the coating, for example by using ultrasonic vibration of the coated device. Another way that acicular particulate could be applied to polyurethane coated medical devices is by blowing the particles through a tube, preferably without using a nozzle, in a manner permitting alignment of the particles in the air stream during the coating process.

Optionally, a surface of the medical device can be coated with a biocompatible polyurethane, and the particulate can be adhered to the polyurethane coating to form a vessel-engaging region in a two-step coating process, or can be incorporated with the polyurethane coating in a one-step coating process. The biocompatible polyurethane coating can comprise a suitable polyurethane/polycarbamate and urea linkages (hereinafter "-C(O)N or CON-type polymers") may be employed. These include CON type polymers that preferably include a soft segment and a hard segment. The segments can be combined as copolymers or as blends. For example, CON type polymers with soft segments such as PTMO, polyethylene oxide, polypropylene oxide, polycarbonate, polyolefin, polysiloxane (i.e. polydimethylsiloxane), and other polyether soft segments made from higher homologous series of diols may be used. Mixtures of any of the soft segments may also be used. The soft segments also may have either alcohol end groups or amine end groups. The molecular weight of the soft segments may vary from about 500 to about 5,000 g/mole. Preferably, the hard segment is formed from a diisocyanate and diamine. The diisocyanate may be represented by the formula OCN-R-NCO, where -R- may be aliphatic, aromatic, cycloaliphatic or a mixture of aliphatic and aromatic moieties. The diamine used as a component of the hard segment includes aliphatic amines, aromatic amines and amines containing both aliphatic and aromatic moieties. The amines may also contain oxygen and/or halogen atoms in their structures. Other applicable biocompatible polyurethanes include those using a polyol as a component of the hard segment. The biocompatible CON type polymers may be modified with cationic, anionic and aliphatic side chains may also be used. Other biocompatible CON type polymers can include polyurethanes having siloxane segments, also referred to as a siloxane-polyurethane.

One particularly preferred biocompatible polyurethane coating is THORALON (THORATEC, Pleasanton, CA), described in U.S. Pat. Application Publication No. 2002/0065552 A1 and U.S. Pat. No. 4,675,361. The biocompatible polyurethane material sold under the tradename THORALON is a polyurethane base polymer (referred to as BPS-215) blended with a siloxane containing surface modifying additive (referred to as SMA-300). The concentration of the surface modifying additive may be in the range of 0.5% to 5% by weight of the base polymer. The SMA-300 component (THORATEC) is a polyurethane comprising polydimethylsiloxane as a soft segment and the reaction product of diphenylmethane diisocyanate (MDI) and 1,4-butanediol as a hard segment. A process for synthesizing SMA-300 is described, for example, in U.S. Pat. Nos. 4,861,830 and 4,675,361. The BPS-215 component (THORATEC) is a segmented polyetherurethane urea containing a soft segment and a hard segment. The soft segment is made of polytetramethylene oxide (PTMO), and the hard segment is made from the reaction of 4,4'-diphenylmethane diisocyanate (MDI) and ethylene diamine (ED).

THORALON coatings can be formed by mixing the polyetherurethane urea (BPS-215) and the surface modifying additive (SMA-300) substance in a solvent. Optionally, the particulate described above can be included with the solvent. The solvent may include dimethyl formamide (DMF), tetrahydrofuran (THF), dimethyacetamide (DMAC), or dimethyl sulfoxide (DMSO), or mixtures thereof. The composition can contain from about less than 1 wt% to about 40 wt% polymer, and different levels of polymer within the range can be used to fine tune the viscosity needed for a given process. The composition can contain less than 5 wt% polymer for some spray application embodiments, such as 0.1-5.0 wt%. For dipping application methods, compositions desirably comprise about 5 to about 25 wt%. The particulates can be mixed into the composition. For example, the mixing can be performed with a spinning blade mixer for about an hour under ambient pressure and in a temperature range of about 18 °C to about 27 °C. The entire composition can be cast as a sheet, or coated onto an article such as a mandrel or a mold. In one example, the composition can be dried to remove the solvent, and then the dried material can be soaked in distilled water to dissolve the particulates, if they comprise a water-soluble material e.g., salt, and leave pores in the material. In another example, the composition can be coagulated in a bath of distilled water. Since the polymer is insoluble in the water, it will rapidly solidify, trapping some or all of the particulates. The particulates can then dissolve from the polymer, leaving pores in the material. It may be desirable to use warm water for the extraction, for example water at a temperature of about 60 °C. The resulting pore diameter can also be substantially equal to the diameter of the salt grains. The resulting void-to-volume ratio, as defined above, can be substantially equal to the ratio of salt volume to the volume of the polymer plus the salt. Formation of porous THORALON is described, for example, in U.S. Pat. Application Publication Nos. 2003/01 14917 A1 and 2003/0149471 A1.

In a second embodiment, the vessel-engaging region is a particulate coating having a plurality of particles attached to a mesh fabric material. The medical device comprises or may be in the form of a mesh fabric film comprising the particulate coating material. The medical device mesh may be formed into one of many geometric shapes. Depending on the application, the mesh may be formed into the shape of a tube or may be a thin, elastic sheet of polymer. Generally, films are less than 5, 4, 3, 2, or 1 mm thick, more preferably less than 0.75 mm, 0.5 mm, 0.25 mm, or, 0.10 mm thick. Films generally are flexible with a good tensile strength (e.g., greater than 50, preferably greater than 100, and more preferably greater than 150 or 200 N/cm²), good adhesive properties (i.e., adheres to moist or wet surfaces), and have controlled permeability. Polymeric mesh films (which may be porous or non-porous) are particularly useful for application to the surface of a device or implant as well as to the surface of tissue, cavity or an organ. Any polymers suitable for preparation of a frame coating above can typically be used to form a mesh film. Film meshes comprising the particulate composition may take a variety of forms including, but not limited to, surgical meshes, membranes (e.g., barrier membranes), surgical sheets, surgical patches, surgical wraps, bandages, liquid bandages, surgical dressings, gauze, fabrics, tapes, surgical membranes, polymer matrices, shells, envelopes, tissue coverings, and other types of surgical matrices, and scaffolds.

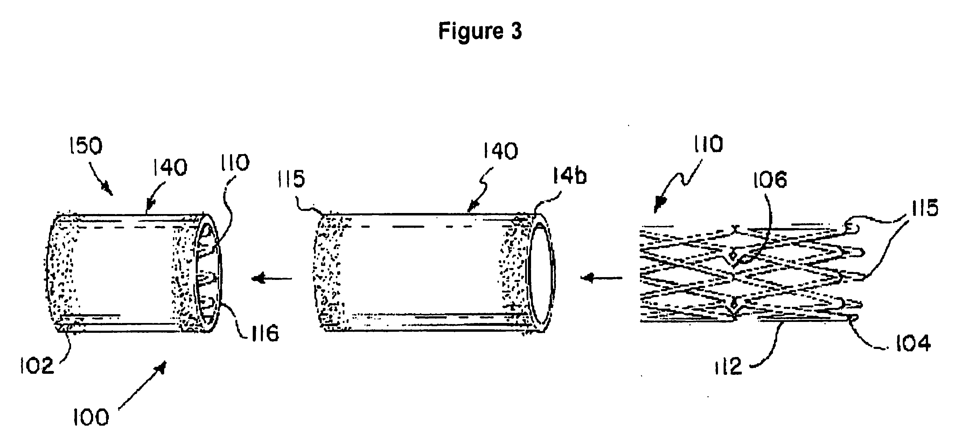

Most preferably, the fabric and particulate composition composite material can be attached to a support frame to form a stent graft, as shown in Figure 3. Figure 3 is an exploded view 100 of a second medical device configured as a stent graft 102. The stent graft 102 comprises a radially expandable frame 110 formed by longitudinally joining eight sinusoidal rings 112 by a plurality of longitudinal connecting struts 106. The distal ring 104 includes a first vessel-engaging region 115 having a particulate coating adapted to engage a graft material. The frame 110 can be secured within the tubular lumen of a flexible material 140 by any suitable means, including stitching, adhesives, or engagement of the flexible material 140 with the first vessel-engaging region's particulate coating 115. The stent graft 102 assembly 1 50 comprises the flexible material 140 secured to the exterior surface of the expandable frame 110 to define a fluid conduit 116.

The flexible material 140 is preferably a mesh composed of a plurality of fibers or filaments (i.e., a fibrous material), where the fibers or filaments are arranged in such a manner (e.g., interwoven, knotted, braided, overlapping, looped, knitted, interlaced, intertwined, webbed, felted, and the like) so as to form a porous structure. Typically, a mesh is a pliable material, such that it has sufficient flexibility to be wrapped around a device. In certain aspects, the mesh may be sufficiently pliable so as to be capable of being wrapped around the external surface of a body passageway or cavity, or a portion thereof. The mesh may be capable of providing support to the structure (e.g., the vessel or cavity wall). In certain aspects, the mesh may be adapted to release an amount of the therapeutic agent. Mesh materials may take a variety of forms. For example, the mesh may be in a woven, knit, or non-woven form and may include fibers or filaments that are randomly oriented relative to each other or that are arranged in an ordered array or pattern. In one embodiment, for example, a mesh may be in the form of a fabric, such as, for example, a knitted, braided, crocheted, woven, non-woven (e.g., a melt-blown or wet-laid) or webbed fabric. In one embodiment, a mesh may include a natural or synthetic biodegradable polymer that may be formed into a knit mesh, a weave mesh, a sprayed mesh, a web mesh, a braided mesh, a looped mesh, and the like. Preferably, a mesh or wrap has intertwined threads that form a porous structure, which may be, for example, knitted, woven, or webbed. The structure and properties of the mesh used in a device depend on the application and the desired mechanical (i.e., flexibility, tensile strength, and elasticity), degradation properties, and the desired loading and release characteristics for the selected therapeutic agent(s).

The mesh should have mechanical properties, such that the device can remain sufficiently strong upon implantation. Factors that affect the flexibility and mechanical strength of the mesh include, for example, the porosity, fabric thickness, fiber diameter, polymer composition (e.g., type of monomers and initiators), process conditions, and the additives that are used to prepare the material. Mesh materials should be sufficiently flexible so as to be capable of conforming to the shape of a device surface or an anatomical surface. In certain cases, the mesh material may be sufficiently flexible so as to be capable of being wrapped around all or a portion of the external surface of a body passageway or cavity. Flexible mesh materials are typically in the form of flexible woven or knitted sheets having a thickness ranging from about 25 micrometers to about 3000 micrometers; preferably from about 50 to about 1000 micrometers. Mesh materials for use in the practice of the invention typically range from about 100 to 400 micrometers in thickness. The mesh may include fibers that are of same dimension or of different dimensions, and the fibers may be formed from the same or different types of biodegradable polymers. Woven materials, for example, may include a regular or irregular array of warp and weft strands and may include one type of polymer in the weft direction and another type (having the same or a different degradation profile from the first polymer) in the warp direction. The degradation profile of the weft polymer may be different than or the same as the degradation profile of the warp polymer. Similarly, knit materials may include one or more types (e.g., monofilament, multi-filament) and sizes of fibers and may include fibers made from the same or from different types of biodegradable polymers.

The particulate coating can be applied to the fabric material in any suitable manner, or can be formed with the material. The particulate may be an integral part of the film or mesh (i.e., may reside within the fibers of the mesh), incorporated directly into the film or mesh or can be incorporated into a carrier (polymeric or non-polymeric), as described above, that is then incorporated into or coated over the film or mesh. Methods for incorporating the particulate compositions onto or into the film or mesh include: (a) affixing (directly or indirectly) to the film or mesh a particulate composition (e.g., by either a spraying process or dipping process as described above, with or without a carrier), (b) incorporating or impregnating into the film or mesh a fibrosing composition (e.g., by either a spraying process or dipping process, with or without a carrier) (c) by coating the film or mesh with a substance such as a hydrogel which can in turn absorb the particulate composition, (d) constructing the film or mesh itself or a portion of the film or mesh with a particulate composition, or (e) by covalently binding the particles directly to the film or mesh surface or to an adhesive that is coated or attached to the film or mesh surface. The coating process can be performed in such a manner as to (a) coat only one surface of the film or mesh or (b) coat all or parts of both sides of the film or mesh. The particulate can be coated onto the entire device or a portion of the device using the polymeric coatings described above. This can be accomplished, for example, by dipping, spraying, electrospinning, painting or by vacuum deposition.

Optionally, a particulate can be combined with a suitable carrier material and applied to the mesh, for example to fill the interstitial spaces within the mesh with the composition comprising the particulate in the carrier material. Particularly preferred carriers include poly(ethylene-co-vinyl acetate), cellulose esters (nitrocellulose), poly(hydroxymethacrylate), poly(methylmethacrylate), poly(ethylene-co-acrylic acid), poly(vinylpyrolidone) polyurethanes (e.g., CHRONOFLEX AL and CHRONOFLEX AR (both from CardioTech International, Inc., Woburn, Mass.) and BIONATE (Polymer Technology Group, Inc., Emeryville, Calif.), poly(D,L-lactic acid) oligomers and polymers, poly(L-lactic acid) oligomers and polymers, poly(glycolic acid), copolymers of lactic acid and glycolic acid, poly(caprolactone), poly(valerolactone), polyanhydrides, poly(anhydride esters), poly(ester-amides), poly(ester-ureas), copolymers of poly(caprolactone) or poly(lactic acid) with a polyethylene glycol (e.g., MePEG), silicone rubbers, poly(styrene)block-poly(isobutylenel-block-poly(styrene), poly(acrylate) polymers, and blends, admixtures, or co-polymers of any of the above. Other preferred polymers include collagen, poly(alkylene oxide)-based polymers, polysaccharides such as hyaluronic acid, chitosan and fucans, and copolymers of polysaccharides with degradable polymers, as well as crosslinked compositions of the above.

Mesh films may be made by several processes, including for example, by casting, and by spraying, or may be formed at the treatment site in situ. For example, a sprayable formulation may be applied onto the treatment site which then forms into a solid film.

Alternatively, the particles may be adhered to the surface of a medical device without an adhesive or other polymer material. For example, the particulate coating may be formed by sputter deposition and/or ion beam deposition methods. Particulate coatings may be formed on implantable medical devices, by depositing a layer on a substrate that is later removed, leaving the layer free standing. The particulate coating formed in the sputter deposition process may be composed of, for example, metals, alloys, compounds, semiconductors, ceramics, and carbon-based materials. The sputtering of a titanium target, for example, will yield a titanium coating. By using more than one sputtering target or a multicomponent target in the deposition process, the coating may be formed from more than one material or element. For example, a target composed of near-stoichiometric nickel titanium (NiTi) may be sputtered to produce a near-stoichiometric NiTi coating or self-supporting structure. Alternatively, to produce oxide, nitride, sulfide or other compounds, a reactive gas may be introduced into the deposition chamber. For example, in order to produce an aluminum oxide partriculate coating, aluminum may be sputtered in an oxygen environment.

### GRAFT MATERIALS

The particulate may be embedded in or coated on a variety of mesh materials to form a medical device, which can further include an implantable support frame attached to the mesh material. Examples of suitable synthetic materials include polymeric materials, such as polypropylene, polyurethane, expanded polytetrafluoroethylene (ePTFE), polyurethane (PU), polyethylene terephthalate (PET), silicone, latex, polyethylene, polypropylene, polycarbonate, nylon, polytetrafluoroethylene, polyimide, polyester, and mixture thereof, or other suitable materials. In one embodiment, the material can comprise a substantially biocompatible material, such as polyester fabrics, polytetrafluoroethylene (PTFE), expanded PTFE, and other synthetic materials known to those of skill in the art.

Examples of suitable natural materials include remodelable materials, such as collagen and extracellular matrix (ECM) material. Small intestine submucosa (SIS) is particularly well-suited as a material, such as to form valve leaflets. Submucosal tissue can be obtained from warm-blooded tissues including the alimentary, respiratory, intestinal, urinary or genital tracts of warm-blooded vertebrates, including without limitation: intestinal submucosa, stomach submucosa, urinary bladder submucosa, and uterine submucosa. Other examples of ECMs are pericardium, stomach submucosa, liver basement membrane, urinary bladder submucosa, tissue mucosa, and dura mater. Information as to submucosa materials useful as ECM materials herein can be found in U.S. Patent Nos. 4,902,508; 5,554,389; 5,993,844; 6,206,931; 6,099,567; and 6,375,989, as well as published U.S. Patent Applications US2004/0180042A1 and US2004/0137042A1. For example, the mucosa can also be derived from vertebrate liver tissue as described in WIPO Publication, WO 98/25637, based on PCT application PCT/US97/22727; from gastric mucosa as described in WIPO Publication, WO 98/26291, based on PCT application PCT/US97/22729; from stomach mucosa as described in WIPO Publication, WO 98/25636, based on PCT application PCT/US97/23010; or from urinary bladder mucosa as described in U.S. Pat. No. 5,554,389.

Preferably, the source tissue for the ECM material is a submucosal tissue, such as tela submucosa, that is disinfected prior to delamination by the preparation disclosed in US Patent Application US2004/0180042A1 by Cook et al., published September 16, 2004. Most preferably, the tunica submucosa of porcine small intestine is processed in this manner to obtain the ECM material. Other disclosures of methods for the isolation of ECM materials include the preparation of intestinal submucosa described in U.S. Pat. No. 4,902,508. Urinary bladder submucosa and its preparation are described in U.S. Pat. No. 5,554,389. Stomach submucosa has also been obtained and characterized using similar tissue processing techniques, for example as described in U.S. patent application US 6099567 titled STOMACH SUBMUCOSA DERIVED TISSUE GRAFT, filed on Dec. 10, 1996.

Any suitable implantable material or portion of an implantable material, including those described above, can serve as the material. The examples recited above provide illustrative examples of some suitable materials that can be selected to provide a material. Other materials known in the art can also be selected to provide a material for some embodiments of the present invention.

### IMPLANTABLE FRAMES

Any suitable implantable frame that can include one or more projections can be used as the Implantable frame in the medical device. The specific implantable frame chosen will depend on several considerations, including the size and configuration of the vessel and the size and nature of the medical device. The frame can perform any desired function, including a stenting function or a valve support function.

An implantable frame that provides a stenting function, i.e., exerts a radially outward force on the interior of the body vessel in which the medical device is implanted, can be used if desired, to provide a stenting functionality at a point of treatment in a body vessel. Any suitable stent configuration can be used as the implantable frame. If a stent is used, the specific stent chosen will depend on several factors, including the vessel in which the medical device is being implanted, the axial length of the treatment site, the number of valves desired in the device, the inner diameter of the body vessel, the delivery method for placing the support structure, and others. Those skilled in the art can determine an appropriate stent based on these and other factors.

The implantable frame can have any suitable configuration and size, depending on several factors, including the desired delivery technique, the nature of the body vessel in which the medical device will be implanted, and the size of the vessel. The implantable frame can be sized so that the expanded configuration is slightly larger in diameter that the inner diameter of the vessel in which the medical device will be implanted. This sizing can facilitate anchoring of the medical device within the body vessel and maintenance of the medical device at a point of treatment following implantation. The support frame can be sized so that the second, expanded configuration is slightly larger in diameter that the inner diameter of the vessel in which the medical device will be implanted. This sizing can facilitate anchoring of the medical device within the body vessel and maintenance of the medical device at a point of treatment following implantation. Preferably, the support frame is configured for implantation in a body vessel having an inner diameter of about 5mm to about 25mm, more preferably about 8mm to about 20mm.

Suitable implantable frames can have a variety of configurations, including braided strands, helically wound strands, ring members, consecutively attached ring members, tube members, and frames cut from solid tubes. Also, suitable frames can have a variety of sizes. The exact configuration and size chosen will depend on several factors, including the desired delivery technique, the nature of the vessel in which the device will be implanted, and the size of the vessel. A frame structure and configuration can be chosen to facilitate maintenance of the device in the vessel following implantation. The implantable frame can be formed in any suitable shape, including a ring, a stent, a tube, or a zig-zag configuration. In one embodiment, the implantable frame can be self-expanding or balloon-expandable.

The implantable frame may be formed from any suitable biocompatible material that allows for desired therapeutic effects upon implantation in a body vessel. Examples of suitable materials include, without limitation, any suitable metal or metal alloy, such as: stainless steels (e.g., 316, 316L or 304), nickel-titanium alloys including shape memory or superelastic types (e.g., nitinol or elastinite); inconel; noble metals including copper, silver, gold, platinum, palladium and iridium; refractory metals including Molybdenum, Tungsten, Tantalum, Titanium, Rhenium, or Niobium; stainless steels alloyed with noble and/or refractory metals; magnesium; amorphous metals; plastically deformable metals (e.g., tantalum); nickel-based alloys (e.g., including platinum, gold and/or tantalum alloys); iron-based alloys (e.g., including platinum, gold and/or tantalum alloys); cobalt-based alloys (e.g., including platinum, gold and/or tantalum alloys); cobalt-chrome alloys (e.g., elgiloy); cobalt-chromium-nickel alloys (e.g., phynox); alloys of cobalt, nickel, chromium and molybdenum (e.g., MP35N or MP20N); cobalt-chromium-vanadium alloys; cobalt-chromium-tungsten alloys; platinum-iridium alloys; platinum-tungsten alloys; magnesium alloys; titanium alloys (e.g., TiC, TiN); tantalum alloys (e.g., TaC, TaN); L605; bioabsorbable materials, including magnesium; or other biocompatible metals and/or alloys thereof. Preferably, the implantable frame comprises a self-expanding nickel titanium (NiTi) alloy material, stainless steel or a cobalt-chromium alloy. The nickel titanium alloy sold under the tradename Nitinol.

Preferably, the frame material is preferably a self-expanding material capable of significant recoverable strain to assume a low profile for delivery to a desired location within a body lumen. After release of the compressed self-expanding resilient material, it is preferred that the frame be capable of radially expanding back to its original diameter or close to its original diameter. Accordingly, some embodiments provide frames made from material with a low yield stress (to make the frame deformable at manageable balloon pressures), high elastic modulus (for minimal recoil), and is work hardened through expansion for high strength. Particularly preferred materials for self-expanding implantable frames are shape memory alloys that exhibit superelastic behavior, i.e., are capable of significant distortion without plastic deformation. Frames manufactured of such materials may be significantly compressed without permanent plastic deformation, i.e., they are compressed such that the maximum strain level in the resilient material is below the recoverable strain limit of the material. Discussions relating to nickel titanium alloys and other alloys that exhibit behaviors suitable for frames can be found in, e.g., U.S. Pat. No. 5,597,378 (Jervis) and WO 95/31945 (Burmeister et al.). A preferred shape memory alloy is Ni-Ti, although any of the other known shape memory alloys may be used as well. Such other alloys include: Au--Cd, Cu--Zn, In--Ti, Cu--Zn--Al, Ti--Nb, Au--Cu--Zn, Cu--Zn--Sn, CuZn--Si, Cu--AI--Ni, Ag--Cd, Cu--Sn, Cu--Zn--Ga, Ni--AI, Fe--Pt, U-Nb, Ti--Pd--Ni, Fe--Mn--Si, and the like. These alloys may also be doped with small amounts of other elements for various property modifications as may be desired and as is known in the art. Nickel titanium alloys suitable for use in manufacturing implantable frames can be obtained from, e.g., Memory Corp., Brookfield, Conn. One suitable material possessing desirable characteristics for self-expansion is Nitinol, a Nickel-Titanium alloy that can recover elastic deformations of up to 10 percent. This unusually large elastic range is commonly known as superelasticity.

The implantable frame can be formed from a variety of medical grade polymers having properties that permit the frame to function as a supporting structure for the remodelable material. In some embodiments, the implantable frame comprises a bioabsorbable or remodelable material.

The implantable frame can comprise a bioabsorbable material that can be degraded and absorbed by the body over time to advantageously eliminate a frame structure from the vessel before, during or after the remodeling process. A number of bioabsorbable homopolymers, copolymers, or blends of bioabsorbable polymers are known in the medical arts. These include, but are not necessarily limited to, polymers and co-polymers comprising a polylactic acid, a polyglycolic acid, a polycaprolactone or derivatives thereof. Suitable bioabsorbable materials for a frame include: poly(epsilon-caprolactone), poly(dimethyl glycolic acid), poly(hydroxy butyrate), poly(p-dioxanone), polydioxanone, PEO/PLA, PLA, poly(lactide-co-glycolide), poly(hydroxybutyrate-co-valerate), poly(glycolic acid-co-trimethylene carbonate), poly(epsilon-caprolactone-co-p-dioxanone), poly-L-glutamic acid or poly-L-lysine, polylactic acid, polylactide, polyglycolic acid, polyglycolide, poly(D,L-lactic acid), L-polylactic acid, poly(glycolic acid), polyhydroxyvalerate, cellulose, chitin, dextran, fibrin, casein, fibrinogen, starch, collagen, hyaluronic acid, hydroxyethyl starch, and gelatin. A frame may also comprise one or more naturally derived bioabsorbable polymers, including modified polysaccharides such as cellulose, chitin, and dextran or modified proteins such as fibrin and casein.

Also provided are embodiments wherein the frame comprises a means for orienting the frame within a body lumen. A frame or delivery device may comprise one or more radiopaque materials to facilitate tracking and positioning of the medical device, which may be added in any fabrication method or absorbed into or sprayed onto the surface of part or all of the medical device. The degree of radiopacity contrast can be altered by implant content. Radiopacity may be imparted by covalently binding iodine to the polymer monomeric building blocks of the elements of the implant. Common radiopaque materials include barium sulfate, bismuth subcarbonate, and zirconium dioxide. Other radiopaque elements include: cadmium, tungsten, gold, tantalum, bismuth, platinum, iridium, and rhodium. In one preferred embodiment, iodine may be employed for its radiopacity and antimicrobial properties. Radiopacity is typically determined by fluoroscope or x-ray film. Various other ways to incorporate radiopaque material in a medical device are provided in copending application 10/787,307, filed February 26, 2004 by Case et al., entitled "Prosthesis Adapted for Placement Under External Imaging,". Imagable markers, including radiopaque material, can be incorporated in any portion of a medical device. For example, radiopaque markers can be used to identify a long axis or a short axis of a medical device within a body vessel. For instance, radiopaque material may be attached to a frame or woven into portions of the valve member material. For example, the frame can comprise a marker, such as a radiopaque portion of the frame that would be seen by remote imaging methods including X-ray, ultrasound, Magnetic Resonance Imaging and the like, or by detecting a signal from or corresponding to the marker. In other embodiments, the delivery device can comprise a frame with indicia relating to the orientation of the frame within the body vessel. In other embodiments, indicia can be located, for example, on a portion of a delivery catheter that can be correlated to the location of the frame within a body vessel.

In one aspect, an implantable medical device is a stent, stent graft or valve implantable in a vessel of a patient and comprising a vessel-engaging region, the medical device being movable between a radially compressed configuration for delivery to a body vessel and a radially expanded position for placement within the body vessel, the medical device adapted to remain in the expanded position in the vessel after a delivery device for the filter is withdrawn from the vessel, the vessel-engaging region of the medical device having a plurality of particles extending from the surface of the medical device, the particles having a geometry and hardness to engage the wall of the body vessel to secure the medical device within the body vessel when released from the delivery device and left within the vessel after withdrawal of the delivery device from the vessel.

### METHODS OF MANUFACTURING

In a first preferred method, methods of applying a coating comprising the particulate to a surface of a medical device are provided. The particulate coating materials can be first mixed with a carrier and a carrier solvent to form a coating solution. The solvent can dissolve the carrier but not the particulate coating particles. The solution comprising the undissolved particles can be coated onto the medical device using any conventional coating processes, for example immersion or dip coating, air knife coating, spray coating or a combination thereof.

Optionally, the device surface can be modified in a manner that promotes the adherence of the particulate coating and/or the carrier. For example, the device surface can be modified prior to dip or spray coating of the particulate coating, by depositing another coating layer comprising a polymer, surface treated by plasma treatment, flame treatment, corona treatment, surface oxidation or reduction, surface etching, mechanical smoothing or roughening, or grafting prior to the coating process. In any one of the above dip coating methods, the surface of the device can be treated with a plasma polymerization method prior to coating, such that a thin polymeric layer is deposited onto the device surface. Examples of such methods include parylene coating of devices and the use of various monomers such hydrocyclosiloxane monomers. Parylene coating may be especially advantageous if the device, or portions of the device, are composed of materials (e.g., stainless steel, cobalt-chromiumnitinol) that do not allow incorporation of the therapeutic agent(s) into the surface layer using one of the above methods. A parylene primer layer may be deposited onto the device using a parylene coater (e.g., PDS 2010 LABCOTER2 from Cookson Electronics) and a suitable reagent (e.g., di-p-xylylene or dichloro-di-p-xylylene) as the coating feed material. Parylene compounds are commercially available, for example, from Specialty Coating Systems, Indianapolis, Ind.), including PARYLENE N (di-p-xylylene), PARYLENE C (a monchlorinated derivative of PARYLENE N, and PARYLENE D, a dichlorinated derivative of Parylene N.J.).

Dip coating is an example of a coating process that can be used to attach a particulate coating to the device. For dip coating, the portion of the medical device to be coated is dipped into the coating solution comprising the particulate coating, a solvent and a solubilized carrier, and then removed. In one embodiment, the solvent does not dissolve the medical device and is not absorbed by the device. Alternatively, the solvent can be selected to be absorbed by the device, thereby swelling the device.

Once dip coated, the device can be immersed, either partially or completely, in the solution for a desired period of time. The rate of immersion into and removal from the solution can be altered (e.g., 0.001 cm per sec to 50 cm per sec). The coated device can be air-dried. The dipping process can be repeated one or more times depending on the specific application. The device can be dried under vacuum to reduce residual solvent levels. This process can result in the particulate coating being adhered to the surface of the device.

The thickness of the resulting particulate coating depends on the concentration of the coating solution and the number of coatings of particulate coating applied. Multiple coating layers may be applied to achieve a desired thickness. The particulate coating preferably has a thickness sufficient to provide a desirable level of durability. Suitable thicknesses are described above. The particulate coating can form a coating over the entire surface of an implantable frame, or only over portions of the frame such as over projections or only the edges of projections from the frame.

A drying phase may be employed after the coating is applied such as air drying, baking, vacuum drying or dehydrating, for example, with a circulating warm gas such as air or nitrogen or other inert gas. Drying of the solution and or coating can carried out in a high-temperature air-bath using a drying oven, drying tunnel, vacuum drier, or any other suitable drying equipment.

Spray coating is another coating process that can be used. In the spray coating process, a solution or suspension of the particulate coating composition, with or without a polymeric or non-polymeric carrier, is nebulized and directed to the device to be coated by a stream of gas. One can use spray devices such as an air-brush (for example models 2020, 360, 175, 100, 200, 150, 350, 250, 400, 3000, 4000, 5000, 6000 from Badger Air-brush Company, Franklin Park, III.), spray painting equipment, TLC reagent sprayers (for example Part # 14545 and 14654, Alltech Associates, Inc. Deerfield, III., and ultrasonic spray devices (for example those available from Sono-Tek, Milton, N.Y.). One can also use powder sprayers and electrostatic sprayers. For air knife coating, the coating is applied, and the excess is "blown off" by a powerful jet from an air knife. For spray coating, the coating solution may be applied to the frame, barb, or portion thereof using any commercial spray coater equipment. Spray coating equipment variables can be manipulated by one skilled in the art to form a suitable particulate coating.

In one embodiment, the solvent is an inert solvent for the device such that the solvent does not dissolve the medical device and is not absorbed by the device to any great extent. Alternatively, solvents that swell the medical device or are absorbed by the device may be used. The device can be held in place or the device can be mounted onto a mandrel or rod that has the ability to move in an X, Y or Z plane or a combination of these planes. Using one of the above described spray devices, the device can be spray coated such that the device is either partially or completely coated with the particulate coating composition. The rate of spraying of the particulate coating composition solution can be altered (e.g., 0.001 ml per sec to 10 ml per sec) to ensure that a good coating of the fibrosis-inducing agent is obtained. The coated device can be air-dried. The spray coating process can be repeated one or more times depending on the specific application. The device can be dried under vacuum to reduce residual solvent levels. This process can result in the fibrosis-inducing agent being coated on the surface of the device.

In one preferred embodiment, a portion of the medical device is dip- or spray-coated with a polyurethane carrier comprising a particulate to for a particulate coating. The polyurethane carrier can be applied with or without the particles. When the polyurethane coating is applied without the particles, the particulate coating is preferably subsequently adhered to the polyurethane coating by placing the particulate in contact with a polyurethane coating. The solution for dip coating is preferably a polyurethane carrier dissolved in a suitable solvent. The dip coating solution preferably comprises the particles, the polyetherurethane urea (BPS-215) and the surface modifying additive (SMA-300) in a suitable solvent that does not dissolve the particulates, preferably DMAC. A solution for forming non-porous THORALON can be made by mixing the polyetherurethane urea (BPS-215) and the surface modifying additive (SMA-300) in a solvent, such as dimethyl formamide (DMF), tetrahydrofuran (THF), dimethyacetamide (DMAC), or dimethyl sulfoxide (DMSO). The solution typically contains between about 0.1 % and 5% by weight SMA-3000 for spray or dip coating solutions. The composition can contain up to about 40 wt% BPS-215 polymer, and different levels of polymer within the range can be used to fine tune the viscosity needed for a given process. Preferably, a solution for spray coating contains less than about 25% by weight of BPS-215 and SMA-3000 in a DMAC solvent, with a viscosity of less than about 2,000 Cp. Typical spray solutions can include a 50:50 weight percentage blend of the DMAC solvent and the pre-mixed solid composition comprising the BPS-215 and SAM-300. Solutions for dip coating can contain more BPS-215, having a viscosity of between about 2,000 and 3,000 Cp. The composition can contain less than 5 wt% polymer for some spray application embodiments. The entire composition can be cast as a sheet, or coated onto an article such as a mandrel or a mold. Preferably, the weight of the particulate added to the solvent is about 5-15 times the amount of solid BPS-215 and SMA-300 added to the solvent, more preferably about 6-12 times. Preferably, the solution and coating surface are at a similar or the same temperature. The mandrel and solution can be maintained at any temperature that maintains the solution in a liquid state with a desired level of viscosity. For THORALON polyureaurethane materials, a mandrel temperature of about 50 to about 60°C is preferred for the dip coating process, preferably about 55°C.

The coating surface can optionally be spun at any suitable rate before, during or after contact with the solution comprising the polyurethane carrier material. The coated medical device can be spun clockwise, counter-clockwise or the rotation can be reversed once or more at any point during the coating or drying process. For THORALON polyurethaneurea carrier materials, the coating surface of a mandrel or assembly can be rotated between about 1 rpm to about 120 rpm in a clockwise direction going into the solution, and a counterclockwise direction during removal from the solution and during drying. The rate of rotation can depend on the viscosity of the solution. Generally, the higher the viscosity of the solution, the faster the mandrel is spun while in contact with the solution, to promote more uniformity in coating thickness over the coating surface. A slowed rotation rate can be employed in a solution with a lower viscosity. The viscosity of the solution of the carrier material solution can be varied, depending on the desired composition of the material. Generally, solution viscosities of between about 200 to 20,000 centipoise are suitable for coating a mandrel or assembly, preferably between about 600 and 1,000 centipoise.

After applying the final coat of the solution, and removal of the coating and removal from the solution, the coated surface of the mandrel, frame or assembly, is preferably dried by removing excess volatile solvent. The coated surface can be dried in a heat chamber, and maintained at a suitable temperature for a suitable period of time to remove excess solvent and dry the coating. The drying temperature can be set suitably high to evaporate excess solvent from the coating, and can depend on the solvent used in the solution. Preferably, the drying temperature is substantially the same as the temperature of the solution and/or the mandrel. For THORALON polyurethaneurea thromboresistant materials dissolved in dimethyacetamide solvent, the final medical device comprising the THORALON material attached to a frame is preferably maintained at a drying temperature of about 40°C to about 60°C for a period of between about 1 minute to about 24 hours to evaporate, more preferably between about 1 hour and 24 hours. Preferably, the coated medical device can be treated to remove any residual solvent, as needed. For example, the medical device can be placed in a water bath to remove trace amounts of DMAC solvent. Preferably, the level of DMAC in the valve leaflet is reduced to about 1090 ppm or lower, more preferably less than about 100 ppm. Residual levels of DMAC in a valve leaflet can be determined by any suitable method, including assays comprising the step of contacting the leaflet with sodium sulfate.

In another embodiment, a solvent casting method can be used to form a carrier polymer, such as polyurethane, into a tubular construct that can incorporate a particulate material. The polyurethane can be coated on the interior surface of the tubular mold by drying a polyurethane solution inside a suitable tubular mold (such as a quartz tube) while' rotating the tubular mold to provide an outer sleeve structure. First, a suitable polyurethane solution, such as porous or non-porous THORALON, is prepared. The solution preferably has a weight ratio of solid (BPS-215 and SMA-300, and optionally containing a salt for forming the porous THORALON material) to DMAC of between about 1:1.5 to about 2:1. Optionally, the solution further comprises an insoluble particles, as described above. The solution can be coated on the inner surface of a glass tubular mold. The coated glass tube can be rotated at about 5 rpm along its longitudinal axis while being heated at a temperature and for a time sufficient to evaporate the solvent (e.g., about 40 °C for about 2 hours). Optionally, hoop members or reinforcing elements (e.g., carbon fibers) can then be placed in contact with the dried coating inside the tube. Another layer of the solution can then be applied to the inside surface of the tube containing the hoop members or reinforcing elements. The glass tube is again heated and rotated to evaporate the solvent, leaving a caste structure having a tubular configuration and incorporating the hoop members or reinforcing elements within the polyurethane wall of the structure. The dried sleeve structure containing the hoop members can be removed from the glass tube and soaked in a warm water bath at a temperature of about 65 °C for about 1 hour, then removed and dried. Optionally, a valve can be radially compressed, deployed and then secured within the lumen of the outer sleeve. The diameter of the tubular mold is preferably less than the maximum diameter of the fully expanded hoop member of valve. Preferably, the valve and hoop members are self-expanding structures that provide an outward radial force to provide shape and stability to the outer sleeve.

Alternatively, the particle coating can be attached directly to the device. This can be accomplished by using an adhesive (e.g., cyanoacrylate, polymer/solvent solution), using a thermal process and or by sewing the fibrosis agent into or onto the device. In one embodiment, the medical device can be coated with particles (irregular, regular, porous, spherical) attached to a mesh of threads, fibers, knits, weaves or electrospun material. For example, a mesh impregnated with particulate coating can be prepared as a knitted, woven or electrospun material that can then be placed on the surface of the device. Sutures and/or an adhesive can then be used to secure the mesh material to the device. The particulate vessel-engaging surface can also be incorporated into the device during or post manufacture of the device.

Particles may also be deposited on the surface of a medical device by sputtering. Sputter deposition entails the transfer of a material from a sputtering target onto a substrate in a controlled partial vacuum environment within a deposition chamber. The material transfer is achieved by bombarding a sputtering target with ions, which effectively knock atoms out of the target, by any suitable method including diode sputtering, radio frequency (rf) sputtering and/or magnetically enhanced sputtering (e.g., dc magnetron sputtering). During deposition, the chamber pressure, sputtering rate and the partial pressure of the process gases are controlled to optimize deposition of the sputtered material onto the substrate. Other parameters influencing the sputter deposition process include the process gas species, the target material, the target geometry, the target-to-substrate distance, the angle of positioning of the substrate with respect to the target, and the substrate temperature. The substrate may be either fixed in place or free to move by, for example, rotation or translation, to facilitate deposition of the target material onto the substrate. The sputtering target may have a circular planar, rectangular planar, triangular planar, cylindrical, conical, ring-like or other shape depending own, for example, the geometry of the substrate and the sputter deposition system used. (Russell J. Hill, Physical Vapor Deposition, 111-190 (The BOC Group,1986)).

Alternatively, ion beam implantation methods are discussed in US Patents 4,693,760 to Piran Sioshansi, 4,743,493 to Piran Sioshansi et al, 4,743,308 to Piran Sioshansi et al, 4,855,026 to Piran Sioshansi, 4,872,922 to Stephen N. Bunker et al, 4,152,478 to Toshinori Takagi, and 4,281,029 to Toshinori Takagi et al. The ion beam deposition of bacterial coatings described in US Patent 5,474,797 to Sioshansi et al. may also be used to deposit coatings of abrasive inorganic metal oxides, such as aluminum oxide. Metallic evaporants include copper, platinum, aluminum, nickel, iridium, silver, gold, and their respective alloys, oxides and compounds may be used in such methods. Aluminum oxide and other abrasive materials can be sputtered onto the metal frames, for example as described in Example 2. We have observed by actual experiment that there is usually no need for an additional adhesive to hold these sputtered particles to the frame.

Other methods include making valves for implantation in a body vessel. In one embodiment, the method comprises the step of attaching a first valve member to a frame. The valve member can be a valve leaflet responsive to the flow of fluid through the frame, and adapted to permit fluid flow through said vessel in a first direction or substantially prevent fluid flow through said vessel in a second, opposite direction. The frame can have a longitudinal axis, a first radial compressibility along a first radial direction that is less than a second radial compressibility along a second radial direction.

### METHODS OF TREATMENT

A medical device can be compressed to a delivery configuration within a retaining sheath that is part of a delivery system, such as a catheter-based system. Upon delivery, the delivery configuration can be expanded, for example, by removing a self-expanding frame, or portion thereof, from the sheath or by inflating a balloon from inside the medical device. The delivery configuration can be maintained prior to deployment of the medical device by any suitable means, including a sheath, a suture, a tube or other restraining material around all or part of the compressed medical device, or other methods. For example, when the medical device is a stent having a particulate coating described above, the method of treatment may involve implanting the stent into the vascular system of a patient and securing the stent within the body vessel by engaging the particulate coating within the wall of the body vessel.

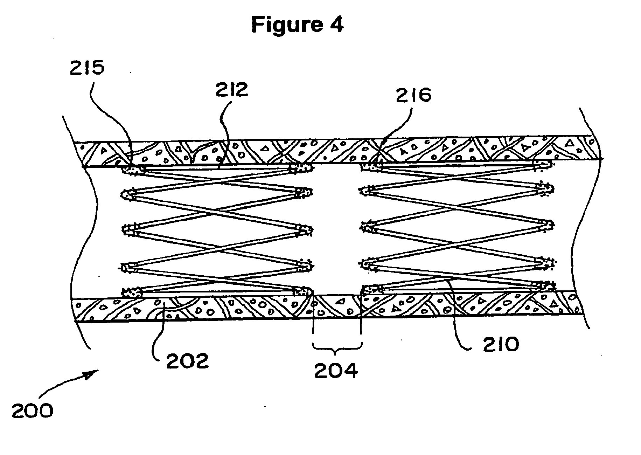

Figure 4 is a longitudinal cross sectional view of a body vessel segment 200 having a pair of the implantable frames 210, 212 (as shown in Figure 1) implanted at a fixed longitudinal distance 204 from each other. The method comprises the steps of endolumenally implanting a first frame 210 comprising a particulate coating 216 on the distal and proximal bends of the first frame 210, endolumenally implanting a second frame 212 comprising a particulate coating 215 on the distal and proximal bends of the second frame 212 at a first distance 204 between the distal end of the first frame 210 and the proximal end of the second frame 212. The particulate coatings 21 5, 216 secure each frame 210, 212 by engaging the wall 202 of the body vessel 200 segment. The medical devices of the embodiments described herein may be oriented in any suitable absolute orientation with respect to a body vessel. The recitation of a "first" direction is provided as an example. Any suitable orientation or direction may correspond to a "first" direction. The medical devices of the embodiments described herein may be oriented in any suitable absolute orientation with respect to a body vessel. For example, the first direction can be a radial direction in some embodiments.

The delivering step can comprise delivery of a medical device comprising a particulate coating by surgical or more preferably by percutaneous delivery techniques known to those skilled in the art. Still other embodiments provide methods of treating a subject, which can be animal or human, comprising the step of providing one or more frames as described herein.

Optionally, the medical device may be configured as an implantable valve comprising a particulate coating on a support frame. Methods for treating certain conditions are also provided, such as venous valve insufficiency, varicose veins, esophageal reflux, restenosis or atherosclerosis. Some embodiments relate to methods of treating venous valve related conditions. A "venous valve related condition" is any condition presenting symptoms that can be diagnostically associated with improper function of one or more venous valves. In mammalian veins, natural valves are positioned along the length of the vessel in the form of leaflets disposed annularly along the inside wall of the vein which open to permit blood flow toward the heart and close to prevent back flow. These natural venous valves act as open to permit the flow of fluid in the desired direction, and close upon a change in pressure, such as a transition from systole to diastole. When blood flows through the vein, the pressure forces the valve leaflets apart as they flex in the direction of blood flow and move towards the inside wall of the vessel, creating an opening therebetween for blood flow. The leaflets, however, do not normally bend in the opposite direction and therefore return to a closed position to restrict or prevent blood flow in the opposite, i.e. retrograde, direction after the pressure is relieved. The leaflets, when functioning properly, extend radially inwardly toward one another such that the tips contact each other to block backflow of blood. Two examples of venous valve related conditions are chronic venous insufficiency and varicose veins.

Methods of treating a venous valve condition can therefore desirably further comprise the step of implanting a valve comprising a particulate coating within a vein or other body vessel. In the condition of venous valve insufficiency, the valve leaflets do not function properly. For example, the vein can be too large in relation to the leaflets so that the leaflets cannot come into adequate contact to prevent backflow (primary venous valve insufficiency), or as a result of clotting within the vein that thickens the leaflets (secondary venous valve insufficiency). Incompetent venous valves can result in symptoms such as swelling and varicose veins, causing great discomfort and pain to the patient. If left untreated, venous valve insufficiency can result in excessive retrograde venous blood flow through incompetent venous valves, which can cause venous stasis ulcers of the skin and subcutaneous tissue. Venous valve insufficiency can occur, for example, in the superficial venous system, such as the saphenous veins in the leg, or in the deep venous system, such as the femoral and popliteal veins extending along the back of the knee to the groin.

The varicose vein condition consists of dilatation and tortuousity of the superficial veins of the lower limb and resulting cosmetic impairment, pain and ulceration. Primary varicose veins are the result of primary incompetence of the venous valves of the superficial venous system. Secondary varicose veins occur as the result of deep venous hypertension which has damaged the valves of the perforating veins, as well as the deep venous valves. The initial defect in primary varicose veins often involves localized incompetence of a venous valve thus allowing reflux of blood from the deep venous system to the superficial venous system. This incompetence is traditionally thought to arise at the saphenofemoral junction but may also start at the perforators. Thus, gross saphenofemoral valvular dysfunction may be present in even mild varicose veins with competent distal veins. Even in the presence of incompetent perforation, occlusion of the saphenofemoral junction usually normalizes venous pressure.

The initial defect in secondary varicose veins is often incompetence of a venous valve secondary to hypertension in the deep venous system. Since this increased pressure is manifested in the deep and perforating veins, correction of one site of incompetence could clearly be insufficient as other sites of incompetence will be prone to develop. However, repair of the deep vein valves would correct the deep venous hypertension and could potentially correct the secondary valve failure. Apart from the initial defect, the pathophysiology is similar to that of varicose veins.

In one preferred embodiment, the medical devices are implanted to treat peripheral vascular disease, for example by implanting the coated medical device in a peripheral artery. Peripheral vascular disease (PVD) is a common condition with variable morbidity affecting mostly men and women older than 50 years. Peripheral vascular disease of the lower extremities comprise a clinical spectrum that goes from asymptomatic patients, to patients with chronic critical limb ischemia (CLI) that might result in amputation and limb loss. Critical limb ischemia is a persistent and relentless problem that severely impairs the patient functional status and quality of life, and is associated with an increased cardiovascular mortality and morbidity. It can present acutely (i.e. distal embolization, external compression, acute thrombosis, etc.) or, in the majority of cases, as chronic CLI. Based on incidence rates extrapolated to today's increasingly aging population, PVD affects as many as 10 million people in the United States (Becker GJ, McClenny TE, Kovacs ME, et al., "The importance of increasing public and physician awareness of peripheral arterial disease," J Vasc Interv Radiol., 13(1):7-11 (Jan 2002)). As the population ages, the family physician will be faced with increasing numbers of patients complaining of symptoms of lower extremity PVD. Nearly one in four of the approximately 60,000 people screened annually through Legs for Life, a nationwide screening program, are determined to be at moderate to high risk of lower extremity PVD and are referred to their primary care physicians for diagnosis (data collected by the Society of Cardiovascular and Interventional Radiology) (Becker GJ, McClenny TE, Kovacs ME, et al., "The importance of increasing public and physician awareness of peripheral arterial disease," J Vasc Interv Radiol., 13(1):7-11 (Jan 2002)).

Methods of treating peripheral vascular disease, including critical limb ischemia, preferably comprise the endovascular implantation of one or more medical devices provided herein. The coated medical device can be implanted in any suitable body vessel. The configuration of the implantable frame can be selected based on the desired site of implantation. For example, for implantation in the superficial artery, popliteal artery or tibial artery, frame designs with increased resistance to crush may be desired. For implantation in the renal or iliac arteries, frame designs with suitable levels of radial force and flexibility may be desired. Preferably, a coated vascular stent is implanted in a non-coronary peripheral artery, such as the iliac or renal arteries.

Methods for delivering a medical device as described herein to any suitable body vessel are also provided, such as a vein, artery, billiary duct, ureteral vessel, body passage or portion of the alimentary canal.

### DEVICE DELIVERY

Preferably, the medical device is implanted in a radially compressed configuration, and expanded at a point of treatment within a body vessel. The overall configuration, cross-sectional area, and length of the frame in the tubular configuration (compressed or expanded) will depend on several factors, including the size and configuration of device, the size and configuration of the vessel in which the device will be implanted, the extent of contact between the device and the walls of the vessel, and the amount of retrograde flow through the vessel that is desired.

Implantable frames or prostheses comprising the implantable frame can be delivered into a body lumen using a system which includes a catheter. In some embodiments, implantable frames can be intralumenally delivered inside the body by a catheter that supports the implantable frame in a compacted form as it is transported to the desired site, for example within a body vessel. Upon reaching the site, the implantable frame can be expanded and securably placed within the body vessel, for example by securably engaging the walls of the body vessel lumen. The expansion mechanism may involve forcing the stent to expand radially outward, for example, by inflation of a balloon formed in the distal portion of the catheter, to inelastically deform the stent and fix it at a predetermined expanded position in contact with the lumen wall. The expansion balloon can then be deflated and the catheter removed. In another technique, the implantable frame is formed of an elastic material that will self-expand after being compacted. During introduction into the body, the implantable frame is restrained in the radially compressed configuration. When the frame has been delivered to the desired site for implantation, the restraint is removed, allowing the implantable frame to self-expand by its own internal elastic restoring force. Once the implantable frame is located at the constricted portion of the lumen, the sheath is removed to expose the stent, which is expanded so it contacts the lumen wall. The catheter is subsequently removed from the body by pulling it in the proximal direction, through the larger lumen diameter created by the expanded prosthesis, which is left in the body.

For example, a method for implanting a stent having an outer surface including a plurality of vessel-engaging particles within the lumen of a body passageway, may include: inserting a catheter delivery system including the stent in a radially compressed configuration within a body vessel, advancing the portion of the catheter delivery system including the stent through a body vessel to a point of treatment, radially expanding the stent within the body vessel at the point of treatment to engage the vessel-engaging particles with the vessel wall so as to implant the stent at the point of treatment and removing the catheter delivery system without the stent from the body vessel.

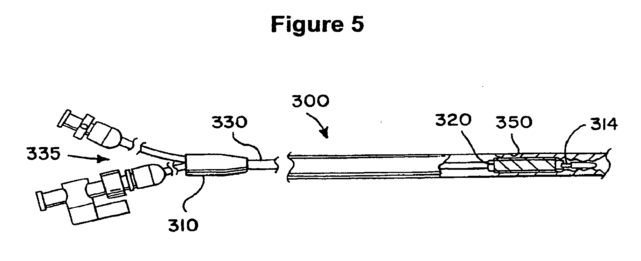

The implantable frames are designed to be percutaneously delivered through a body lumen to a target site. The target site may be, for example, a location in the venous system adjacent to an insufficient venous valve. The implantable frames may be delivered, for example, on their own or as part of an implantable prosthetic valve. Figure 5 illustrates a delivery system 300. The delivery system 300 includes a catheter 310 having a distal end 314. A balloon 320 is positioned on the distal end 314 of the catheter 310. A connector assembly 330 is disposed at the proximal end 335 of the catheter 310 and is adapted to facilitate expansion of the balloon 320 as is known in the art. The connector assembly 330 provides access to an interior lumen of the catheter 310 to provide access to the balloon 320, and possibly a wire guide (not illustrated) or other conventional component. A balloon expandable frame 350 is disposed on the distal end 314 of the catheter 310. The expandable frame 350 surrounds the balloon 320 and is initially, prior to placement in a body vessel, in its unexpanded state. This positioning allows the balloon 320, upon inflation, to expand the expandable frame 350 into its expanded state. The medical device shown in Figure 5 can be deployed by expansion of the balloon 320 within a body vessel. The catheter is positioned at a point of treatment within a body vessel. The balloon 260 is then inflated to expand the medical device to the expanded configuration. Upon inflation of the balloon, the particulate coating contacts the interior surface of the body vessel. Subsequently, the balloon can be deflated and the delivery catheter removed from the body vessel along the wire guide.

Alternatively, the support frame can comprise a self-expanding material such as nitinol, coated with a metallic bioabsorbable material. A medical device comprising a self-expanding support frame can be deployed from a catheter that includes a moveable sheath containing the support frame instead of a balloon. The sheath can be longitudinally translated with respect to the medical device, away from the distal end of the delivery catheter. When the sheath no longer covers the medical device, the self-expanding support frame can radially expand to contact the inner wall of the body vessel, where the medical device can be maintained by the outward force exerted by the frame or by barbs or perforations in the exterior surface of the medical device.

An appropriately sized delivery catheter can be selected by one skilled in the art for a given application. For example, some embodiments can be delivered using a delivery catheter selected from one or more delivery catheter sizes from the group consisting of: 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30 french (F) delivery catheters, or increments of 0.1 F therebetween. In some embodiments, a delivery catheter sized between 1 and 25 F, or preferably between about 1.5F and 5F can be used, preferably a 1.8F (0.60mm), 2.0F (0.66mm), 2.3F (0.75 mm), 2.6F (0.85mm), 2.7 F (0.9mm), 2.9F (0.95mm), or 3.3 11.10 mm) delivery catheters.

In some embodiments, the implantable frames impart radially outward directed force during deployment, whether self-expanding or radially-expandable. The radially outward directed force can serve to hold the body lumen open against a force directed radially inward, as well as preventing restriction of the passageway through the lumen by intimal flaps or dissections generated by, such as prior balloon angioplasty. Another function of the radially outward directed force can also fix the position of the stent within the body lumen by intimate contact between the stent and the walls of the lumen. Preferably, the outwardly directed forces do not traumatize the lumen walls.

In one aspect a method of implanting a medical device in a patient's body is provided, wherein the method includes: providing a medical device with a surface having a vessel-engaging section formed from a plurality of inorganic particles and a radially expandable support frame, providing a delivery catheter containing the medical device in a radially compressed configuration having a first diameter, inserting the medical device in the radially compressed configuration adjacent to point of treatment (e.g., a stenosis in the body vessel) so that the vessel-engaging section faces toward the wall of the body vessel, deploying the medical device from the delivery catheter so the medical device radially expands to an expanded configuration with the vessel-engaging section in vessel-engaging contact with the wall of the body vessel, the expanded configuration having a diameter larger than the first diameter with at least a portion of the particles on the surface of the vessel-engaging portion embedded within the body vessel wall, and withdrawing the delivery catheter from the vessel leaving the medical device secured within the vessel in the expanded diameter placement configuration with the vessel with the vessel-engaging portion engaging and retaining a wall of the body vessel.

In another aspect, a method of implanting one or more stents each having a particulate vessel-engaging region, and each optionally including associated graft material, within one or more points of treatment in a body vessel includes: inserting a wire guide to guide a stent (optionally with an associated first graft) to the first vessel region, inserting a first delivery sheath containing the first stent (optionally with the associated first graft) over the wire guide to the point of treatment, and removing the first delivery sheath to enable the first stent (optionally with the associated first graft) to expand against the wall of the first point of treatment until the vessel-engaging region is secured to the wall of the body vessel at the first point of treatment by embedding at least a portion of a plurality of particles within the vessel-engaging region within the wall of the body vessel.

In yet another aspect, a method of implanting a medical device within a peripheral artery or within the deep or superficial venous system comprises: inserting a wire guide into a body vessel to guide a medical device to a point of treatment within a body vessel, subsequent to inserting the wire(s) guide inserting the medical device including a vessel-engaging region including a plurality of vessel-engaging projections over the wire guide to a point of treatment within the body vessel, expanding the medical device at the point of treatment against the body vessel until the vessel-engaging region retains the wall of the body vessel, and removing the wire guide from the body vessel while retaining the medical device at the point of treatment.

The implantable frames can be placed in any medically appropriate location for a given application. For example, in some embodiments, the implantable frame can serve as part of a venous valve prosthetic and be implanted in the femoral vein, including at the proximal (groin), mid (mid section) or distal (adjacent to the knee) portions of the vein.

### EXAMPLES

### Example 1

A particular embodiment was made that comprised a nitinol self-expanding stent (ZILVER Stent, Cook Incorporated, Bloomington, IN) onto which needle-like, polymeric particles were attached. Specifically, the particles had barbs on one end that resembled the barbs on burdock seeds. The non-barbed ends of these particles were adhered to the stent with cyanoacrylate adhesive. A small amount of instant cyanoacrylate adhesive (LOCTITE 401, Loctite Corp, Rocky Hill, Conn.) was applied to each particle, which was held in contact with the abluminal (exterior) surface of the stent near the distal or proximal end until the particle was adhered to the stent (e.g., about 1 minute). Each particle was about 2 mm in length, oriented with the barb radiating away from the lumen of the stent. About 20-30 particles were adhered to each end of the abluminal (exterior) surface of the stent with a density of about 1 particle per 3-4 mm² of abluminal stent surface area. A further embodiment could comprise particles with fishhook-like barbs on one end and made of metal or a high durometer polymer.

### Example 2

Another embodiment was fabricated by sputtering aluminum oxide onto nitinol stents and valve frames. A particulate coating of a metal or metal oxide particles may be formed by sputtering methods, such as ion implantation. For example, an aluminum oxide (Al₂O₃) surface layer was deposited on the abluminal surface of a nitinol vascular stent (ZILVER Stent, Cook Incorporated, Bloomington, IN).

The particulate coating was prepared by ion beam deposition to from the aluminum oxide coating, which may be performed according to methods described in US 5,123,924. For example, the ion implantation process may be performed in an ion implantation chamber having a high current ion implantation device (e.g., Varian Extrion 200kV Implanter) with an aluminum base plate. The ion beam preferably incorporates one of a group of elemental species, including nitrogen, oxygen, carbon, titanium, beryllium, neon, krypton, helium, phosphorus, argon and other noble gases. Aluminum oxide possesses a considerably lower sputtering coefficient, i.e., between about 0.06 and 0.09 at 50 keV for N + than does pure aluminum, whose sputtering coefficient is about 0.3 for 50 keV N +. Ion beam deposition is preferably performed at a suitable vacuum, such as less than 10-6 torr and is preferably generated with an oil-free pump. During deposition, the ion beam power density acting on the surfaces of the workpieces perferably does not exceed about 6.0 watt/cm² and preferably is about 1.0 watt/cm². Consequently, the peak ion beam power density of an 80 keV beam preferably does not exceed about twelve microamperes per square centimeter. Another discussion of ion beam deposition methods is found in US 5,474,797.

The resulting aluminum oxide on these parts was bonded securely and had a maximum size range below .001 inch (25.4 micrometers). The concentration of particles exceeded 10,000 per square centimeter.

## Claims

1. A vessel-engaging medical device (10) comprising a radially expandable medical device (12) adapted for implantation in a body vessel, the medical device defining a substantially tubular lumen and having an exterior surface facing radially opposite the tubular lumen, wherein at least a portion of the exterior surface comprises a vessel-engaging surface (15) formed by a plurality of inorganic particles (30) attached to the exterior surface to form the vessel-engaging surface with an average roughness of between 10 nm and 100 micrometers, the inorganic particles (30) consisting of one or more materials selected from the group consisting of: metals, metal alloys, inorganic oxides, silicon carbide, hydroxyapatite, silicon dioxide, iron oxide, alumina, zirconia, silicon carbide, alumina zirconia, emery, stainless steel, cobalt-chromium, nickel-titanium alloy, and tantalum.

2. The medical device of claim 1 wherein the vessel-engaging surface (15) has a maximum roughness of less than 25 micrometers and a concentration of inorganic particles (30) of at least 10,000 per square centimeter of the vessel-engaging surface (15).

3. The medical device of claim 2, wherein the inorganic particles (30) consist of aluminum oxide.

4. The medical device of claim 1, further **characterized by**
(a) the medical device (12) being configured as a tubular metallic vascular stent or stent graft with the vessel-engaging surface (15) formed on a portion of the exterior surface of the medical device;
(b) the vessel-engaging surface (15) being free of a polymer and having an average roughness of between 1 and 25 micrometers, a maximum roughness of less than 25 micrometers and a concentration of inorganic particles (30) of at least 10,000 per square centimeter of the vessel-engaging surface; and
(c) the vessel-engaging surface consisting of a plurality of aluminum or aluminum oxide particles.

5. The medical device of claim 1, further **characterized by**
(a) the medical device (12) being configured as a tubular metallic vascular stent or stent graft with the vessel-engaging surface (15) formed on a portion of the exterior surface of the medical device;
(b) the vessel-engaging surface (15) consisting of a polymer carrier in contact with the inorganic particles and the exterior surface of the medical device, the vessel-engaging surface (15) having an average roughness of between 1 and 100 micrometers and a concentration of inorganic particles (30 of 7.75 to 15.5 particles per square centimeter of the vessel-engaging surface; and
(c) the inorganic particles (30) consisting of a cellulose-containing biopolymer.

6. The medical device of claim 1, where the inorganic particles (30) have an acicular shape and preferably a portion that is hooked or barbed.

7. A method of adapting a radially expandable medical device (12) for secure implantation within a body vessel by forming a vessel-engaging surface (15) on the medical device, the method comprising the step of adhering a plurality of inorganic particles (30) to a surface of the medical device so that said inorganic particles form the vessel-engaging surface (15) with an average surface roughness of between 10 nm and 100 micrometers the inorganic particles (30) consisting of one or more materials selected from the group consisting of: metals, metal alloys, inorganic oxides, silicon carbide, hydroxyapatite, silicon dioxide, iron oxide, alumina, zirconia, silicon carbide, alumina zirconia, emery, stainless steel, cobalt-chromium, nickel-titanium alloy, and tantalum.

8. The method of Claim 7, where the step of adhering the inorganic particles (30) to the surface of the medical device (12) includes sputter deposition or ion beam implantation of the inorganic particles.

9. The method of claim 7, where the step of adhering the inorganic particles (30) to the surface of the medical device (12) includes applying a polymer, preferably a cyanoacrylate polymer, to a surface of the medical device (12) and contacting the polymer on the surface with the inorganic particles (30) to adhere the inorganic particles to the polymer.

10. The method of at least one of claims 7-9, where the medical device (12) is a stent or stent graft having a substantially tubular shape when radially expanded with a first vessel-engaging region (15) on the exterior surface of the tubular shape, where the vessel-engaging region (15) includes an average of 7.75 to 12.4 particles per square centimeter.

11. The method of at least one of claims 7 - 10, where the inorganic particles (30) further comprise a biodegradable material.

12. The method of at least one of claims 7-11, where the inorganic particles (30) have an acicular shape and preferably a portion that is hooked or barbed.

13. The method of at least one of claims 7-12, where the medical device (12) is configured as a vascular stent comprising a proximal hoop member and a first vessel-engaging region on the proximal hoop member, the proximal hoop member joined to a distal hoop member with a second vessel-engaging region on the distal hoop member or as a stent graft (102) comprising a support frame (110) attached to a tubular graft material (140) having an exterior surface and an interior surface, the vessel-engaging region (15, 115) being disposed on the exterior surface of the graft material.

14. The method of at least one of claims 7-13, where the vessel-engaging surface (15) has a maximum roughness of less than 25 micrometers and a concentration of inorganic particles (30) of at least 10,000 per square centimeter in the vessel-engaging surface (15).

## Patentansprüche

1. In ein Gefäß eingreifende medizinische Vorrichtung (10), die eine radial expandierbare medizinische Vorrichtung (12) zur Implantation in ein Körpergefäß umfasst, wobei die medizinische Vorrichtung ein im Wesentlichen röhrenförmiges Lumen definiert und eine Außenseite aufweist, die radial entgegengesetzt zum röhrenförmigen Lumen weist, worin zumindest ein Teil der Außenseite eine Gefäßeingriffsfläche (15) umfasst, die von einer Vielzahl von anorganischen Teilchen (30), die an der Außenseite befestigt sind, um die Gewebeeingriffsfläche mit einer durchschnittlichen Rauhigkeit von 10 nm bis 100 Mikrometer zu formen, geformt wird, wobei die anorganischen Teilchen (30) aus einem oder mehr Materialien bestehen, die aus der folgenden Gruppe ausgewählt sind: Metalle, Metalllegierungen, anorganische Oxide, Silikonkarbid, Hydroxylapatit, Silikondioxid, Eisenoxid, Aluminiumoxid, Zirkoniumdioxid, Silikonkarbid, Aluminiumoxidzirkondioxid, Korund, Edelstahl, Kobalt-Chrom, Nickel-Titan-Legierung und Tantal.

2. Medizinische Vorrichtung nach Anspruch 1, worin die Gewebeeingriffsfläche (15) eine maximale Rauhigkeit von unter 25 Mikrometer und eine Konzentration von anorganischen Teilchen (30) von zumindest 10.000 pro Quadratzentimeter der Gewebeeingriffsfläche (15) aufweist.

3. Medizinische Vorrichtung nach Anspruch 2, worin die anorganischen Teilchen (30) aus Aluminiumoxid bestehen.

4. Medizinische Vorrichtung nach Anspruch 1, ferner **dadurch gekennzeichnet, dass**
(a) die medizinische Vorrichtung (12) als röhrenförmiger metallischer Gefäßstent oder als Gefäß-Stentgraftprothese konfiguriert ist, wobei die Gewebeeingriffsfläche (15) auf einem Teil der Außenseite der medizinischen Vorrichtung geformt ist;
(b) die Gewebeeingriffsfläche (15) kein Polymer enthält und eine durchschnittliche Rauhigkeit zwischen 1 und 25 Mikrometer, eine maximale Rauhigkeit von unter 25 Mikrometer und eine Konzentration von anorganischen Teilchen (30) von zumindest 10.000 pro Quadratzentimeter der Gewebeeingriffsfläche aufweist; und
(c) die Gewebeeingriffsfläche aus einer Vielzahl von Aluminium- oder Aluminiumoxid-Teilchen besteht.

5. Medizinische Vorrichtung nach Anspruch 1, ferner **dadurch gekennzeichnet, dass**
(a) die medizinische Vorrichtung (12) als röhrenförmiger metallischer Gefäßstent oder als Gefäß-Stentgraftprothese konfiguriert ist, wobei die Gewebeeingriffsfläche (15) auf einem Teil der Außenseite der medizinischen Vorrichtung geformt ist;
(b) die Gewebeeingriffsfläche (15) aus einem Polymerträger in Kontakt mit den anorganischen Teilchen und der Außenseite der medizinischen Vorrichtung besteht, wobei die Gefäßeingriffsfläche (15) eine durchschnittliche Rauhigkeit zwischen 1 und 100 Mikrometer und eine Konzentration von anorganischen Teilchen (30) von 7,75 bis 15,5 Teilchen pro Quadratzentimeter der Gewebeeingriffsfläche aufweist; und
(c) die anorganischen Teilchen (30) aus einem cellulosehaltigen Biopolymer bestehen.

6. Medizinische Vorrichtung nach Anspruch 1, worin die anorganischen Teilchen (30) eine nadelförmige Gestalt und vorzugsweise einen mit Haken oder Widerhaken versehenen Abschnitt aufweisen.

7. Verfahren zur Anpassung einer radial expandierbaren medizinischen Vorrichtung (12) zur sicheren Implantation in einem Körpergefäß durch Bildung einer Gewebeeingriffsfläche (15) der medizinischen Vorrichtung, wobei das Verfahren den Schritt der Anheftung einer Vielzahl von anorganischen Teilchen (30) auf einer Oberfläche der medizinischen Vorrichtung umfasst, so dass die anorganischen Teilchen die Gewebeeingriffsfläche (15) mit einer durchschnittlichen Oberflächenrauhigkeit von 10 nm bis 100 Mikrometer formen, wobei die anorganischen Teilchen (30) aus einem oder mehr Materialien bestehen, die aus der folgenden Gruppe ausgewählt sind: Metalle, Metalllegierungen, anorganische Oxide, Silikonkarbid, Hydroxylapatit, Silikondioxid, Eisenoxid, Aluminiumoxid, Zirkoniumdioxid, Silikonkarbid, Aluminiumoxidzirkondioxid, Korund, Edelstahl, Kobalt-Chrom, Nickel-Titan-Legierung und Tantal.

8. Verfahren nach Anspruch 7, worin der Schritt der Anheftung der anorganischen Teilchen (30) an der Oberfläche der medizinischen Vorrichtung (12) Sputterabscheidung oder Ionenstrahlimplantation der anorganischen Teilchen aufweist.

9. Verfahren nach Anspruch 7, worin der Schritt der Anheftung der anorganischen Teilchen (30) an der Oberfläche der medizinischen Vorrichtung (12) Aufbringen eines Polymers, vorzugsweise eines Cyanoacrylat-Polymers, auf eine Oberfläche der medizinischen Vorrichtung (12) und Inberührungbringen des Polymers auf der Oberfläche mit den anorganischen Teilchen (30) zur Anheftung der anorganischen Teilchen an dem Polymer umfasst.

10. Verfahren nach mindestens einem der Ansprüche 7-9, worin die medizinische Vorrichtung (12) ein Stent oder eine Stentgraftprothese ist, der bzw. die eine im Wesentlichen röhrenförmige Gestalt aufweist, wenn er/sie in einer ersten Gewebeeingriffsregion (15) auf der Außenseite der röhrenförmigen Gestalt radial expandiert ist, wobei die Gewebeeingriffsregion (15) durchschnittlich 7,75 bis 12,4 Teilchen pro Quadratzentimeter aufweist.

11. Verfahren nach mindestens einem der Ansprüche 7-10, worin die anorganischen Teilchen (30) ferner ein biologisch abbaubares Material umfassen.

12. Verfahren nach mindestens einem der Ansprüche 7-11, worin die anorganischen Teilchen (30) eine nadelförmige Gestalt und vorzugweise einen mit Haken oder Widerhaken versehenen Abschnitt aufweisen.

13. Verfahren nach mindestens einem der Ansprüche 7-12, worin die medizinische Vorrichtung (12) als Gefäßstent mit einem proximalen Reifenelement und einer ersten Gefäßeingriffsregion auf dem proximalen Reifenelement, wobei das proximale Reifenelement mit einem distalen Reifenelement mit einer zweiten Gewebeeingriffsregion auf dem distalen Reifenelement verbunden ist, oder als Stentgraftprothese (102) mit einem Stützrahmen (110), der an einem röhrenförmigen Graftprothesenmaterial (140) mit einer Außenseite und einer Innenseite befestigt ist, konfiguriert ist, wobei die Gewebeeingriffsregion (15, 115) auf der Außenseite des Graftprothesenmaterials angeordnet ist.

14. Verfahren nach mindestens einem der Ansprüche 7-13, worin die Gewebeeingriffsfläche (15) eine maximale Rauhigkeit von weniger als 25 Mikrometer und eine Konzentration von anorganischen Teilchen (30) von zumindest 10.000 pro Quadratzentimeter in der Gewebeeingriffsfläche (15) aufweist.

## Revendications

1. Dispositif médical d'engagement de vaisseau (10) comprenant un dispositif médical radialement expansible (12) adapté pour implantation dans un vaisseau corporel, le dispositif médical définissant une lumière sensiblement tubulaire et ayant une surface extérieure orientée radialement face à la lumière tubulaire, où au moins une partie de la surface extérieure comprend une signal d'engagement de vaisseau (15) formée par une pluralité de particules inorganiques (30) fixées à la surface extérieure pour former la surface d'engagement de vaisseau avec une rugosité moyenne comprise entre 10 nm et 100 micromètres, les particules inorganiques (30) étant constituées d'un ou plusieurs matériaux choisis dans le groupe constitué de : métaux, alliages métalliques, oxydes inorganiques, carbure de silicium, hydroxyapatite, dioxyde de silicium, oxyde de fer, alumine, oxyde de zirconium, carbure de silicium, oxyde d'aluminium-zirconium, émeri, acier inoxydable, cobalt-chrome, alliage nickel-titane, et tantale.

2. Dispositif médical de la revendication 1, dans lequel la surface d'engagement de vaisseau (15) a une rugosité maximale de moins de 25 micromètres et une concentration de particules inorganiques (30) d'au moins 10 000 par centimètre carré de la surface d'engagement de vaisseau (15).

3. Dispositif médical de la revendication 2, dans lequel les particules inorganiques (30) sont constituées d'oxyde d'aluminium.

4. Dispositif médical de la revendication 1, **caractérisé en outre en ce que**
(a) le dispositif médical (12) est configuré sous la forme d'une endoprothèse vasculaire métallique tubulaire ou d'une greffe d'endoprothèse avec la surface d'engagement de vaisseau (15) formée sur une partie de la surface extérieure du dispositif médical ;
(b) la surface d'engagement de vaisseau (15) est exempte d'un polymère et ayant une rugosité moyenne comprise entre 1 et 25 micromètres, une rugosité maximale de moins de 25 micromètres et une concentration de particules inorganiques (30) d'au moins 10 000 par centimètre carré de la surface d'engagement de Vaisseau ; et
(c) la surface d'engagement de vaisseau est constituée d'une pluralité de particules d'aluminium ou d'oxyde d'aluminium.

5. Dispositif médical de la revendication 1, **caractérisé en outre en ce que**
(a) le dispositif médical (12) est configuré sous la forme d'une endoprothèse vasculaire métallique tubulaire ou d'une greffe d'endoprothèse avec la surface d'engagement de vaisseau (15) formée sur une partie de la surface extérieure du dispositif médical ;
(b) la surface d'engagement de vaisseau (15) est constituée d'un support de polymère en contact avec les particules inorganiques et la surface extérieure du dispositif médical, la surface d'engagement de vaisseau (15) ayant une rugosité moyenne comprise entre 1 et 100 micromètres et une concentration de particules inorganiques (30 de 7,75 à 15,5 particules par centimètre carré de la surface d'engagement de Vaisseau ; et
(c) les particules inorganiques (30) constituées d'un biopolymère contenant de la cellulose.

6. Dispositif médical de la revendication 1, dans lequel les particules inorganiques (30) ont une forme aciculaire et de préférence une partie en forme de crochet ou de barbule.

7. Procédé d'adaptation d'un dispositif médical radialement expansible (12) pour implantation fixe dans un vaisseau corporel par formation d'une surface d'engagement de vaisseau (15) sur le dispositif médical, le procédé comprenant l'étape d'adhésion d'une pluralité de particules inorganiques (30) à une surface du dispositif médical de sorte que lesdites particules inorganiques forment la surface d'engagement de vaisseau (15) avec une rugosité de surface moyenne comprise entre 10 nm et 100 micromètres, les particules inorganiques (30) étant constituées d'un ou plusieurs matériaux choisis dans le groupe constitué de : métaux, alliages métalliques, oxydes inorganiques, carbure de silicium, hydroxyapatite, dioxyde de silicium, oxyde de fer, alumine, oxyde de zirconium, carbure de silicium, oxyde d'aluminium-zirconium, émeri, acier inoxydable, cobalt-chrome, alliage nickel-titane, et tantale.

8. Procédé de la revendication 7, dans lequel l'étape d'adhésion des particules inorganiques (30) à la surface du dispositif médical (12) comprend le dépôt par pulvérisation ou l'implantation de faisceau ionique des particules inorganiques.

9. Procédé de la revendication 7, dans lequel l'étape d'adhésion des particules inorganiques (30) à la surface du dispositif médical (12) comprend l'application d'un polymère, de préférence un polymère de cyanoacrylate, à une surface du dispositif médical (12) et la mise en contact du polymère sur la surface avec les particules inorganiques (30) pour faire adhérer les particules inorganiques au polymère.

10. Procédé d'au moins une des revendications 7 à 9, dans lequel le dispositif médical (12) est une endoprothèse ou une greffe d'endoprothèse ayant une forme sensiblement tubulaire lorsqu'il est radialement expansé avec une première région d'engagement de vaisseau (15) sur la surface extérieure de la forme tubulaire, où la région d'engagement du vaisseau (15) comprend une moyenne de 7,75 à 12,4 particules par centimètre carré.

11. Procédé d'au moins une des revendications 7 à 10, dans lequel les particules inorganiques (30) comprennent en outre un matériau biodégradable.

12. Procédé d'au moins une des revendications 7 à 11, dans lequel les particules inorganiques (30) ont une forme aciculaire et de préférence une partie en forme de crochet ou de barbule.

13. Procédé d'au moins une des revendications 7 à 12, où le dispositif médical (12) est configuré sous la forme d'une endoprothèse vasculaire comprenant un élément de cerceau proximal et une première région d'engagement de vaisseau sur l'élément de cerceau proximal, l'élément de cerceau proximal assemblé à un élément de cerceau distal avec une deuxième région d'engagement de vaisseau sur l'élément de cerceau distal ou sous la forme d'une greffe d'endoprothèse (102) comprenant un cadre de support (110) attaché à un matériau de greffe tubulaire (140) ayant une surface extérieure et une surface intérieure, la région d'engagement de vaisseau (15, 115) étant disposée sur la surface extérieure du matériau de greffe.

14. Procédé d'au moins une des revendications 7 à 13, où la surface d'engagement de vaisseau (15) a une rugosité maximale de moins de 25 micromètres et une concentration de particules inorganiques (30) d'au moins 10 000 par centimètre carré dans la surface d'engagement de vaisseau (15).
